# EUROPÄISCHE PATENTSCHRIFT

(11) **EP 4 106 822 B1**
(45) Veröffentlichungstag und Bekanntmachung des Hinweises auf die Patenterteilung: **17.04.2024**
(21) Anmeldenummer: 21703437.0
(22) Anmeldetag: 04.02.2021
(51) Int. Cl.: A61L 9/01, C11D 3/30, C11D 3/50, A61L 2/00, C11D 3/28, A61L 9/04

(54) **ZUSAMMENSETZUNG, ENTHALTEND MIKOVERKAPSELTE SCHLECHTGERUCHSBEKÄMPFUFNGSMITTEL UND IHRE VERWENDUNG ZUR VERMINDERUNG VON FEHLGERÜCHEN**
COMPOSITION COMPRISING MICRO-ENCAPSULATED MALODOR CONTROL AGENTS AND THEIR USE FOR REDUCING MALODORS
COMPOSITION COMPRENANT DES AGENTS ANTI-MAUVAISES ODEURS MICRO-ENCAPSULÉS ET LEUR UTILISATION POUR RÉDUIRE LES MAUVAISES ODEURS

(30) Priorität: 19.02.2020 EP 20158274
(43) Veröffentlichungstag der Anmeldung: 28.12.2022
(73) Patentinhaber: Henkel AG & Co. KGaA, 40589 Düsseldorf (DE)
(72) Erfinder: BAUER, Andreas, 41564 Kaarst (DE); WEYHE, Marc, 47802 Krefeld (DE); GUILMET, Erwan, 40593 Düsseldorf (DE); SORG, Rainer, 47906 Kempen (DE); SCHEUER, Madeline, 47447 Moers (DE)
(86) Internationale Anmeldenummer: PCT/EP2021/052621
(87) Internationale Veröffentlichungsnummer: WO 2021/165046

(56) Entgegenhaltungen:
- EP-A1- 2 204 156
- EP-A1- 3 045 518
- EP-A1- 3 197 421
- DE-A1-102009 026 856
- DE-A1-102012 201 424
- DE-A1-102017 111 444

## Beschreibung

Die vorliegende Erfindung betrifft eine Zusammensetzung umfassend Mikrokapseln enthaltend mindestens ein Geruchsneutralisierungsmittel sowie Mittel enthaltend eine derartige Zusammensetzung. Weiterhin richtet sich die vorliegende Erfindung auf die Verwendung derartiger Zusammensetzungen oder Mittel zur Verminderung von Fehlgerüchen und auf Verfahren zur Behandlung einer Oberfläche, zur Behandlung der Raumluft, oder zum Waschen und/oder Pflegen von Textilien unter Anwendung derartiger Zusammensetzungen oder Mittel.

Die marktgängigen Wasch- und Reinigungsmittel sowie Kosmetikprodukte werden in aller Regel parfümiert. Seitens der Verbraucher wird sowohl eine ausreichende Eigenbeduftung des Produktes als auch eine Verminderung von "schlechten" Nebengerüchen gewünscht. Weiter werden auch langanhaltende und frische Parfümierungen gewünscht. Schlecht- bzw. Fehlgerüche gehen von bestimmten geruchsaktiven Verbindungen aus, wie z.B. bestimmten Ketonen oder Aldehyden, z.B. 2,4-Decadienal, insbesondere kurzkettigen Aldehyden. DE102009026856 A1 offenbart (substituiertes) 2-Amino-1,3 propandiol zum Abbau von Fehlgerüchen.

1-Aza-3,7-dioxabicyclo[3.3.0]octan-Verbindungen sind unter anderem aus EP3045518 A1 und DE102012201424 A1 bekannt. EP3197421 B1 offenbart mikroverkapselte Duftstoffe zur Beseitigung von Fehlgerüchen.

Aufgabe der vorliegenden Erfindung war es, dem Verbraucher eine Möglichkeit zu geben, eine Verminderung von Fehlgerüchen, insbesondere solchen, die auf Aldehyde und/oder Ketone zurückzuführen sind, herbeizuführen.

Es wurde nun gefunden, dass diese Aufgabe durch Formulierungen enthaltend verkapselte Geruchsneutralisierungsmittel gelöst wird.

In einem ersten Aspekt richtet sich die vorliegende Erfindung daher auf eine Zusammensetzung umfassend Mikrokapseln umfassend mindestens einen Kern und eine Schale, wobei der Kern mindestens ein Geruchsneutralisierungsmittel enthält ausgewählt aus einer 1-Aza-3,7-dioxabicyclo[3.3.0]octan-Verbindung der allgemeinen Formel (II) 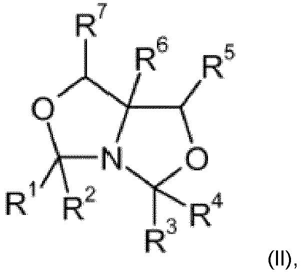 wobei, in den Verbindungen gemäß Formel (II), die Reste R¹, R², R³ und R⁴, jeweils unabhängig voneinander, für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben, und die Reste R⁵, R⁶ und R⁷, jeweils unabhängig voneinander, für H oder einen Kohlenwasserstoffrest stehen, der acyclisch oder cyclisch, substituiert oder unsubstituiert, verzweigt oder unverzweigt, gesättigt oder ungesättift sein kann;
dadurch gekennzeichnet, dass, in den Verbindungen der Formel (II),
- die Reste R², R⁴, R⁵, und R⁷ für H stehen und die Reste R¹ und R³ jeweils unabhängig für einen C₆₋₂₄-Kohlenwasserstoffrest stehen und R⁶ für H oder einen C₁₋₂₄-Kohlenwasserstoffrest steht; oder
- die Reste R⁵ und R⁷, jeweils unabhängig voneinander, für Wasserstoff oder einen C₁₋₆-Kohlenwasserstoffrest, der ggf. substituiert sein kann, vorzugsweise C₁₋₃- Kohlenwasserstoffrest stehen, insbesondere sind R⁵ und R⁷ jeweils Wasserstoff oder jeweils ein Methyl- oder Ethylrest; und/oder
- der Rest R⁶ für H, einen Methyl-, Ethyl- oder Hydroxymethylrest steht; und/oder
- die Reste R² und R⁴ jeweils für H stehen; und/oder
- die Reste R², R⁴, R⁵ und R⁷ jeweils für H stehen, der Rest R⁶ für H, einen Methyl-, Ethyl- oder Hydroxymethylrest steht, die Reste R¹ und R³, jeweils unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt, gesättigt oder ungesättigt sein kann; oder
- die Reste R², R⁴, R⁵, R⁶ und R⁷ für H stehen und die Reste R¹ und R³ jeweils unabhängig für einen C₅₋₂₄-Kohlenwasserstoffrest stehen.

In einem weiteren Aspekt richtet sich die vorliegende Erfindung auf ein Mittel umfassend mindestens eine Zusammensetzung, wie hierin beschrieben, wobei es sich bei dem Mittel um ein Wasch-, Reinigungs- oder Behandlungsmittel handelt.

In noch einem weiteren Aspekt richtet sich die vorliegende Erfindung auf die Verwendung einer hierin beschriebenen Zusammensetzung oder eines hierin beschriebenen Mittels zur Verminderung von Fehlgerüchen.

In einem letzten Aspekt richtet sich die vorliegende Erfindung schließlich auch auf ein Verfahren zur Behandlung einer Oberfläche, zur Behandlung der Raumluft, oder zum Waschen und/oder Pflegen von Textilien, dadurch gekennzeichnet, dass in mindestens einem Verfahrensschritt eine hierin beschriebene Zusammensetzung und/oder ein hierin beschriebenes Mittel angewendet wird.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-%, jeweils bezogen auf das Gesamtgewicht der entsprechenden Zusammensetzung. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens ein", wie hierin verwendet, bezieht sich auf 1 oder mehr, beispielsweise 1, 2, 3, 4, 5, 6, 7, 8, 9 oder mehr.

"Ungefähr", wie hierin in Bezug auf Zahlenwerte verwendet, bedeutet den entsprechenden Wert ±10%, vorzugsweise ±5%.

Wie überraschenderweise gefunden wurde, kann die Verminderung, d.h. im Kontext der vorliegenden Erfindung Neutralisierung von Schlecht- bzw. Fehlgerüchen, d.h. unerwünschten Gerüchen, durch die nachfolgend beschriebenen, verkapselten Geruchsneutralisierungsmittel im Endprodukt, d.h. beispielsweise in einem Universalwaschmittel, im Vergleich zu Endprodukten mit Geruchsneutralisierungsmittel, allerdings in unverkapselter Form, deutlich erhöht werden. Die Verbesserung drückt sich dabei nicht nur durch eine verstärkte (direkte) Fehlgeruchsneutralisierung, wie nachfolgend definiert, sondern darüber hinaus durch eine verbesserte Langlebigkeit der Fehlgeruchsneutralisierung aus. Insbesondere wurde gefunden, dass die Neutralisierung von Fehlgerüchen, die auf Ketone und/oder Aldehyde, insbesondere kurzkettige Aldehyde zurückzuführen sind, verbessert werden kann.

Im Kontext der vorliegenden Erfindung bedeutet das Neutralisieren von Fehlgerüchen nicht nur das Überdecken von Fehlgerüchen, sondern das Deaktivieren der Fehlgeruch-auslösenden Stoffe. Deaktivierung bedeutet hier, dass der Fehlgeruch zumindest vermindert und insbesondere sogar gänzlich beseitigt, also ausgelöscht wird. Immer wenn ein Fehlgeruch vorliegt, muss es ein Objekt geben, von dem dieser Fehlgeruch ausgeht, oder einen Raum oder ein System, in welchem der Fehlgeruch wahrnehmbar ist. An diesem Objekt oder diesem Raum kann eine erfindungsgemäße Zusammensetzung, beispielsweise als Additiv/Bestandteil eines Wasch-, Reinigungs- oder Behandlungsmittels, wie hierin definiert, angewendet werden.

Demgemäß ist ein erster Gegenstand der vorliegenden Erfindung eine Zusammensetzung umfassend Mikrokapseln umfassend mindestens einen Kern und eine Schale, wobei der Kern mindestens ein Geruchsneutralisierungsmittel enthält.

Geruchsneutralisierungsmittel, welche zur Verwendung im Kontext der vorliegenden Erfindung geeignet sind, sind im Stand der Technik prinzipiell bekannt und schließen alljene Verbindungen ein, welche die Neutralisierung von Fehlgerüchen, wie voranstehend definiert, beispielsweise haftend an einem Textilstück, bewirken.

Die Geruchsneutralisierungsmittel werden, im Kontext der vorliegender Erfindung, ausgewählt aus der Gruppe bestehend aus 1-Aza-3,7-dioxabicyclo[3.3.0]octan-Verbindungen der allgemeinen Formel (II) 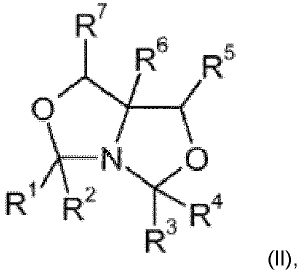 wobei, in den Verbindungen gemäß Formel (II), die Reste R¹, R², R³ und R⁴, jeweils unabhängig voneinander, für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben, und die Reste R⁵, R⁶ und R⁷, jeweils unabhängig voneinander, für H oder einen Kohlenwasserstoffrest stehen, der acyclisch oder cyclisch, substituiert oder unsubstituiert, verzweigt oder unverzweigt, gesättigt oder ungesättift sein kann;
dadurch gekennzeichnet, dass, in den Verbindungen der Formel (II),
- die Reste R², R⁴, R⁵, und R⁷ für H stehen und die Reste R¹ und R³ jeweils unabhängig für einen C₆₋₂₄-Kohlenwasserstoffrest stehen und R⁶ für H oder einen C₁₋₂₄-Kohlenwasserstoffrest steht; oder
- die Reste R⁵ und R⁷, jeweils unabhängig voneinander, für Wasserstoff oder einen C₁₋₆-Kohlenwasserstoffrest, der ggf. substituiert sein kann, vorzugsweise C₁₋₃- Kohlenwasserstoffrest stehen, insbesondere sind R⁵ und R⁷ jeweils Wasserstoff oder jeweils ein Methyl- oder Ethylrest; und/oder
- der Rest R⁶ für H, einen Methyl-, Ethyl- oder Hydroxymethylrest steht; und/oder
- die Reste R² und R⁴ jeweils für H stehen; und/oder
- die Reste R², R⁴, R⁵ und R⁷ jeweils für H stehen, der Rest R⁶ für H, einen Methyl-, Ethyl- oder Hydroxymethylrest steht, die Reste R¹ und R³, jeweils unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt, gesättigt oder ungesättigt sein kann; oder
- die Reste R², R⁴, R⁵, R⁶ und R⁷ für H stehen und die Reste R¹ und R³ jeweils unabhängig für einen C₅₋₂₄-Kohlenwasserstoffrest stehen.

Es wird angenommen, dass die durch Hydrolyse der Verbindung der Formel (II) freigesetzten Aminoalkohole eine Deaktivierung von Stinkstoffen bewirken. Die bei der Hydrolyse der Verbindungen der Formel (II) ebenfalls freigesetzten Riechstoffaldehyde und/oder -ketone tragen dabei zusätzlich zur Überdeckung des Fehlgeruchs bei. Erfindungsgemäß wird somit ein deutlicher Abbau bis hin zur Auslöschung von Fehlgerüchen ermöglicht.

Alle Kohlenwasserstoff-Reste im Sinne der Erfindung können grundsätzlich acyclisch oder cyclisch, substituiert oder unsubstituiert, verzweigt oder unverzweigt sowie gesättigt oder ungesättigt sein. Die Kohlenwasserstoff-Reste im Sinne der Erfindung können grundsätzlich Heteroatome, wie z.B. Stickstoff-, Sauerstoff- oder Schwefel-Atome umfassen. Bevorzugt mit Blick auf R⁵, R⁶ und R⁷ sind jeweils acyclische, unverzweigte Kohlenwasserstoffreste, die ggf. substituiert sein können. Geeignete Substituenten sind z.B. Hydroxy-, Alkoxy-, Amino- oder Halogen-Gruppen. Der Kohlenwasserstoffrest kann beispielsweise lineares oder verzweigtes, substituiertes oder unsubstituiertes Alkyl mit 1 bis 10 Kohlenstoffatomen sein, z.B. mit 1 bis 6 Kohlenstoffatomen oder 1, 2, 3, 4, 5 oder 6 Kohlenstoffatomen, Als substituierte Varianten kommen auch die entsprechenden Alkoxyreste oder Hydroxy-, Amino- oder Haloalkylgruppen in Frage.

Wie bereits definiert, stehen, in Formel (II), die Reste R¹, R², R³, R⁴jeweils unabhängig voneinander für Reste, die in einer Verbindung der allgemeinen Formel R¹-C(=O)-R² oder R³-C(=O)-R⁴ einen Riechstoff-Aldehyd mit mindestens 6 Kohlenstoffatomen oder ein Riechstoff-Keton mit mindestens 6 Kohlenstoffatomen ergeben.

Vorzugsweise liegen maximal in einem der Strukturelemente -CR¹R² oder -CR³R⁴ Reste R¹ und R² oder R³ und R⁴ vor, die in einer Verbindung der allgemeinen Formel R¹-C(=O)-R² oder R³-C(=O)-R⁴ ein Riechstoffketon ergeben. Insbesondere liegen in beiden Strukturelementen -CR¹R² und -CR³R⁴ Reste R¹ und R² sowie R³ und R⁴ vor, die in einer Verbindung der allgemeinen Formel R¹-C(=O)-R² sowie R³-C(=O)-R⁴ jeweils ein Riechstoffaldehyd ergeben, insbesondere das gleiche Riechstoffaldehyd. Geeignete Riechstoffketone und -aldehyde sind dem Fachmann bekannt und werden beispielshaft im Folgenden aufgelistet.

Der Riechstoff-Aldehyd ist gemäß einer bevorzugten Ausführungsform der Erfindung ausgewählt aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-Isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-timethyl-2(1)-cyclo-hexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-car-boxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pen-tenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarbox-aldehyd, 7-Hydroxy-3,7-dimethyl-octanal, trans-4-Decenal, 2,6-Nonadienal, para-Tolyl-acetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Di-methyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propyl-bicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

Das Riechstoff-Keton ist gemäß einer bevorzugten Ausführungsform der Erfindung ausgewählt aus Methyl-beta-naphthylketon, Moschusindanon (1,2,3,5,6,7-Hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-Pentamethylhept-3-en-2-on), Fenchon, alpha-lonon, beta-lonon, gamma-Methyl-lonon, Fleuramon (2-Heptylcyclopen-tanon), Dihydrojasmon, cis-Jasmon, iso-E-Super (1-(1,2,3,4,5,6,7,8-Octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3-methyl-5-propyl-2-cyclohexenon), 6-Isopropyldecahydro-2-naphton, Dimethyloctenon, Frescomenthe (2-Butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-Benzodioxol-5-yl) butan-2-on), Hexalon (1-(2,6,6-Trimethyl-2-cyclohexene-1-yl)-1,6-heptadien-3-on), Isocyclemon E (2-Acetonaphthon-1,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541-91-3), Neobutenon (1-(5,5-Dimethyl-1-cyclohexenyl)pent-4-en-1-on), Plicaton (CAS 41724-19-0), Velouton (2,2,5-Trimethyl-5-pentylcyclopentan-1-on), 2,4,4,7-Tetramethyl-oct-6-en-3-on sowie Tetrameran (6,10-Dimethylundecen-2-on).

R¹ und R³ stehen gemäß einer bevorzugten Ausführungsform der Erfindung, unabhängig voneinander, vorteilhafterweise jeweils für einen C₆₋₂₄-Kohlenwasserstoffrest, vorzugsweise C₇₋₂₄-Kohlenwasserstoffrest, wobei der Kohlenwasserstoffrest acyclisch oder cyclisch, substituiert oder unsubstituiert, verzweigt oder unverzweigt sowie gesättigt oder ungesättigt sein kann.

Bezogen auf R⁶ sind bevorzugte Reste C₁₋₁₆-Kohlenwasserstoffreste, insbesondere C₁₋₁₂-Kohlenwasserstoffreste, weiter bevorzugt C₁₋₆- Kohlenwasserstoffreste, am meisten bevorzugt C₁₋₃-Kohlenwasserstoffreste. Vorzugsweise handelt es sich um unverzweigte, acyclische Alkylreste. Sie können auch substituiert sein. Es kann sich z.B. um Mono- oder Dihydroxyalkylreste handeln, die anstelle der Hydroxylgruppen oder zusätzlich auch eine Aminogruppe aufweisen können. Sofern die Kohlenwasserstoffreste durch -O- unterbrochen sind, handelt es sich vorzugsweise um Strukturelemente der Formel -CH₂-CH₂-O- oder -CH₂-CH(CH₃)-O-. Derartige Verbindungen sind in einfacher Weise durch Alkoxylierung der entsprechenden Hydroxyverbindungen zugänglich.

Geeignete Oxazolidine gemäß der allgemeinen Formel (II) sind demnach z.B. 1-Aza-3,7-dioxa-2,8-diheptyl-bicyclo[3.3.0]octan, 1-Aza-3,7-dioxa-2,8-diheptyl-5-methyl-bicyclo[3.3.0]octan, 1-Aza-3,7-dioxa-2,8-diheptyl-5-hydroxymethyl-bicyclo[3.3.0]octan, 1-Aza-3,7-dioxa-2,8-diheptyl-5-ethyl-bicyclo[3.3.0]octan ,1-Aza-3,7-dioxa-2,8-dioctyl-bi-cyclo[3.3.0]octan, 1-Aza-3,7-dioxa-2,8-dioctyl-5-methyl-bicyclo[3.3.0]octan, 1-Aza-3,7-dioxa-2,8-dioctyl-5-hydroxymethyl-bicyclo[3.3.0]octan sowie 1-Aza-3,7-dioxa-2,8-dioctyl-5-ethyl-bicyclo[3.3.0]octan.

Die Verbindungen der allgemeinen Formel (II) sind insbesondere erhältlich durch Umsetzung von Verbindungen der allgemeinen Formel (I) 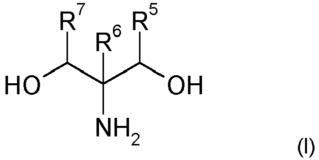 mit Verbindungen der allgemeinen Formeln R¹-C(=O)-R² und R³-C(=O)-R⁴ unter Ringschluss. Eine geeignete Substanz gemäß Formel (I) ist z.B. das 2-Aminopropan-1 ,3-diol. Für die Reste R¹ bis R⁷ gilt jeweils das zuvor bereits Geschriebene. Die allgemeinen Formeln R¹-C(=O)-R² und R³-C(=O)-R⁴ repräsentieren im Sinne der Erfindung ganz allgemein Riechstoff-Aldehyde oder Riechstoff-Ketone. Riechstoff-Aldehyde sind diejenigen Riechstoffe, welche chemisch ein Aldehyd sind und welche vorteilhafterweise beim Menschen ein insbesondere angenehmes Geruchsempfinden auslösen. Riechstoff-Ketone sind diejenigen Riechstoffe, welche chemisch ein Keton sind und welche vorteilhafterweise beim Menschen ein insbesondere angenehmes Geruchsempfinden auslösen. Besonders geeignete Riechstoff-Aldehyde sowie Riechstoff-Ketone werden weiter oben exemplarisch aufgeführt. Zur Veranschaulichung seien zwei Beispiele gegeben. Beispielsweise steht bei dem Riechstoff-Aldehyd Octanal entsprechend der allgemeinen Formel R¹-C(=O)-R² der Rest R¹ für einen Heptylrest (also für CH₃-(CH₂)₆)- und der Rest R² steht für Wasserstoff oder umgekehrt. Beispielsweise steht bei dem Riechstoff-Keton Methylnonylketon entsprechend der allgemeinen Formel R¹-C(=O)-R² der Rest R¹ für einen Methylrest und der Rest R² steht für einen Nonylrest (also CH₃-(CH₂)₈-) oder umgekehrt.

Als Riechstoff-Aldehyde und/oder Riechstoff-Ketone können grundsätzlich alle üblichen Riechstoff-Aldehyde und/oder Riechstoff-Ketone eingesetzt werden, die insbesondere zur Herbeiführung eines angenehmen Geruchsempfindens beim Menschen eingesetzt werden. Solche Riechstoff-Aldehyde und/oder Riechstoff-Ketone sind dem Fachmann bekannt und auch in der Patentliteratur, beispielsweise in US 2003/0158079 A1, Absätze [0154] und [0155] beschrieben.

Zur Herstellung der erfindungsgemäß einzusetzenden Verbindungen der allgemeinen Formel (II) kann also eine Verbindung der allgemeinen Formel (I) mit Aldehyden, Ketonen oder Mischungen von Ketonen und Aldehyden unter Ringschluß umgesetzt werden. Gemäß einigen Ausführungsformen der Erfindung leiten sich die Verbindungen der allgemeinen Formel (II) von einem Molekül der allgemeinen Formel (I) und zwei Aldehydmolekülen, welche gleich oder verschieden sein können, oder einem Aldehydmolekül und einem Ketonmolekül ab. Bei der Umsetzung von geringeren als stöchiometrischen Mengen an Aldehyden und/oder Ketonen liegen im Produktgemisch auch monocyclische Verbindungen vor. Der Anteil an bicyclischen Verbindungen zu monocyclischen Verbindungen kann aber in einfacher Weise durch Auswahl des Molverhältnisses zwischen Aldehyd/Keton und der Verbindung der allgemeinen Formel (I) eingestellt werden.

Die Umsetzung wird dabei vorzugsweise in einem geeigneten Lösungsmittel oder *in situ* durchgeführt. Geeignete Lösungsmittel sind beispielsweise aromatenhaltige Kohlenwasserstoffe wie Toluol. Die Umsetzung wird dabei vorzugsweise bei einer Temperatur im Bereich von 80 bis 150 °C, besonders bevorzugt 100 bis 140 °C durchgeführt. Beispielsweise wird die Verbindung der allgemeinen Formel (I) unter Stickstoffatmosphäre zusammen mit dem gewünschten Keton und/oder Aldehyd im Lösungsmittel vorgelegt. Sodann wird das Reaktionsgemisch erhitzt. Häufig wird sodann unter Rückfluss am Wasserabscheider erhitzt. Das erhaltene Umsetzungsprodukt wird nach üblichen Verfahren isoliert und gegebenenfalls gereinigt. In WO2007/087977 A1, auf welche hiermit Bezug genommen wird, wird die Herstellung von Verbindungen der allgemeinen Formel (II) auch anhand von Synthesebeispielen detailliert beschrieben.

In verschiedenen Ausführungsformen der vorliegenden Erfindung kann das mindestens eine Geruchsneutralisierungsmittel, wie voranstehend definiert, in Kombination mit einer oder mehrerer Verbindungen der allgemeinen Formel (IV) eingesetzt werden 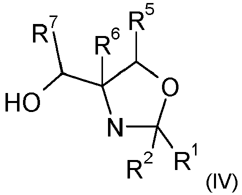 wobei, in den Verbindungen der allgemeinen Formel (IV),
R¹, R² unabhängig voneinander für Reste stehen, die in einer Verbindung der allgemeinen Formel R¹-C(=O)-R² einen Duftstoff-Aldehyd mit mindestens 6 Kohlenstoffatomen oder ein Duftstoff-Keton mit mindestens 6 Kohlenstoffatomen ergeben, und R⁵, R⁶, R⁷ unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann.

In verschiedenen Ausführungsformen der Erfindung kann das mindestens eine Geruchsneutralisierungsmittel, d.h. mindestens eine 1-Aza-3,7-dioxabicyclo[3.3.0]octan-Verbindung der allgemeinen Formel (II) zusammen mit Riechstoffen eingesetzt werden, wobei die 1-Aza-3,7-dioxabicyclo[3.3.0]octan-Verbindung in Gewichtsmengen, bezogen auf die Riechstoffgesamtmenge, vorzugsweise im Bereich von 1:100 bis 100:1, insbesondere von 10:1 bis 1:50, beispielsweise im Bereich von 10:1 bis 1:10 oder 10:1 bis 1:2 oder 5:1 bis 1:1 eingesetzt wird.

In verschiedenen Ausführungsformen ist das Geruchsneutralisierungsmittel im Wesentlichen ohne zusätzliches freies Parfüm bzw. Duftstoff in der Kapsel enthalten. Da das Geruchsneutralisierungsmittel eine Verbindung der Formel (II) ist, kann die Mikrokapsel zusätzlich zu den Verbindungen der Formel (II) auch die entsprechenden Freisetzungsprodukte, d.h. den freigesetzten Duftstoff und die korrespondieren Verbindung der Formel (I) enthalten. Diese können spontan entstehen. Auch in derartigen Ausführungsformen kann es vorteilhaft sein, dass die Mikrokapsel keinen anderen als den aus den Verbindungen der Formel (II) freigesetzten Duftstoff enthält.

In verschiedenen Ausführungsformen ist das mindestens eine Geruchsneutralisierungsmittel in einer Menge von ungefähr 0,1 bis ungefähr 40 Gew.-%, vorzugsweise ungefähr 0,1 bis ungefähr 20 Gew.-%, jeweils bezogen auf das Gesamtgewicht der Zusammensetzung, in dieser enthalten.

Erfindungsgemäß ist das mindestens eine Geruchsneutralisierungsmittel in der erfindungsgemäßen Zusammensetzung in Mikrokapseln eingekapselt enthalten.

"Mikrokapsel", wie hierin verwendet, bezieht sich auf Kapseln mit Kern-Schale Morphologie im Mikrometermaßstab, die eine Kapselhülle aufweisen, welche einen Kern vollständig einschließt. Erfindungsgemäß ist das mindestens eine Geruchsneutralisierungsmittel Bestandteil des Mikrokapselkerns. Das Gewichtsverhältnis zwischen Schale und Kern liegt üblicherweise im Bereich von 5:95 bis 30:70, vorzugsweise im Bereich von 10:90 bis 20:80, beispielsweise 15:85. Der "Kern" umfasst hierbei den gesamten von der Schale umschlossenen Inhalt, d.h. das Geruchsneutralisierungsmittel und alle etwaigen zusätzlichen Bestandteile.

"Vollständig einschließt" oder "vollständig umgibt", wie hierin in Bezug auf die Mikrokapseln verwendet, bedeutet, dass der Kern vollständig von der Hülle umgeben ist, d.h. insbesondere nicht derart in eine Matrix eingebettet ist, dass er an einer Stelle freiliegt. Es ist ferner bevorzugt, dass die Kapselhülle derart beschaffen ist, dass die Freisetzung des Inhalts kontrolliert wird, d.h. der Inhalt nicht unabhängig von einem Freisetzungsreiz spontan unkontrolliert freigesetzt wird. Aus diesem Grund ist die Kapselhülle vorzugsweise im Wesentlichen undurchlässig für den verkapselten Inhalt. "Im Wesentlichen undurchlässig", wie in diesem Kontext verwendet, bedeutet, dass der Inhalt der Kapsel bzw. einzelne Inhaltsstoffe nicht spontan die Hülle durchdringen können, sondern die Freisetzung nur durch Öffnen der Kapsel oder optional auch über einen über einen längeren Zeitraum ablaufenden Diffusionsprozess erfolgen kann. Der Kern kann fest, flüssig und/oder gasförmig sein, ist aber vorzugsweise fest und/oder flüssig. Die Mikrokapseln sind vorzugsweise im Wesentlichen sphärisch und weisen Durchmesser im Bereich von 0,01 bis 1000 µm, insbesondere 0,1 bis 500 µm auf. Kapselhülle und Kapselkern bestehen aus unterschiedlichen Materialien, insbesondere ist die Kapselhülle bei Standardbedingungen (20°C, 1013 mbar) vorzugsweise fest, der Kern vorzugsweise fest und/oder flüssig, insbesondere flüssig.

Als Kapselmaterial für im Kontext der vorliegenden Erfindung geeignete Mikrokapseln können ganz allgemein z. B. hochmolekulare Verbindungen tierischer oder pflanzlicher Herkunft, z. B. Eiweißverbindungen (Gelatine, Albumin, Casein), Cellulose-Derivate (Methylcellulose, Ethylcellulose, Celluloseacetat, Cellulosenitrat, Carboxymethylcellulose), sowie synthetische Polymere (z. B. Polyamide, Polyolefine, Polyester, Polyurethane, Epoxidharze, Silikonharze und Kondensationsprodukte von Carbonyl- und NH-Gruppen-haltigen Verbindungen) verwendet werden. Konkret kann das Schalenmaterial beispielsweise ausgewählt werden aus Polyacrylaten; Polyethylen; Polyamiden; Polystyrolen; Polyisoprenen; Polycarbonaten; Polyestern; Polyharnstoffen; Polyurethanen; Polyolefinen; Polysacchariden; Epoxidharzen; Vinylpolymeren; Harnstoff vernetzt mit Formaldehyd oder Glutaraldehyd; Melamin vernetzt mit Formaldehyd; Gelatine-Polyphosphat-Koazervaten, optional vernetzt mit Glutaraldehyd; Gelatine-Gummi Arabicum Koazervaten; Silikonharze; mit Polyisocyanaten umgesetzten Polyaminen; mittels freier Radikalpolymerisation polymerisierter Acrylatmonomere; Seide; Wolle; Gelatine; Cellulose; Proteinen; und Mischungen und Copolymeren der vorgenannten. Besonders bevorzugt sind Polyacrylate, Polyethylen, Polyamide, Polystyrole, Polyisoprene, Polycarbonate, Polyester, Polyharnstoffe, Polyurethane, Polyolefine, Epoxidharze, Vinylpolymere und Harnstoff und/oder Melamin vernetzt mit Formaldehyd oder Glutaraldehyd. In verschiedenen Ausführungsformen kann es bevorzugt sein, dass das Kapselmaterial bioabbaubar ist.

Zur Herstellung geeigneter Mikrokapseln sind prinzipiell die bekannten Mikroverkapselungsverfahren geeignet, bei denen z. B. die Einkapselung der einzukapselnden Phase durch Umhüllung mit filmbildenden Polymeren (wie z. B. zuvor genannt), die sich nach Emulgierung und Koazervation oder Grenzflächenpolymerisation auf dem einzuhüllenden Material niederschlagen, erfolgt.

Erfindungsgemäß umfasst die einzukapselnde Phase mindestens ein Geruchsneutralisierungsmittel, wie voranstehend definiert. Weiterhin kann es sich, gemäß einiger Ausführungsformen, bei der einzukapselnden Phase auch um eine Vorteilsmittelzusammensetzung handeln, welche erfindungsgemäß mindestens ein Geruchsneutralisierungsmittel umfasst und vorteilhaft mindestens ein weiteres Vorteilsmittel, beispielsweise eine Parfümölzusammensetzung, wie nachfolgend definiert, enthält.

Die Kapseln können die verkapselten Vorteilsmittel über verschiedene Mechanismen freisetzen. In verschiedenen Ausführungsformen der vorliegenden Erfindung können z.B. Kapseln, die eine mechanisch stabile Kapselhülle aufweisen, die aber dann aufgrund eines oder mehrerer Umwelteinflüsse, wie Änderung der Temperatur oder der lonenstärke oder des pH-Wertes des umgebenden Mediums, für die enthaltenen Mittel durchlässig wird, verwendet werden. Möglich sind auch stabile Kapselwandmaterialien, durch die das mindestens eine Vorteilsmittel, d.h., im Kontext der vorliegenden Erfindung, das mindestens eine Geruchsneutralisierungsmittel, sowie ggf. weitere Vorteilsmittel, mit der Zeit hindurchdiffundieren kann/können. Die Kapseln können das mindestens eine enthaltene Vorteilsmittel vorzugsweise bei Änderung des pH-Wertes oder der lonenstärke der Umgebung, bei Änderung der Temperatur, bei Einwirkung von Licht, durch Diffusion und/oder bei mechanischer Beanspruchung freisetzen.

In einer bevorzugten Ausführungsform der vorliegenden Erfindung sind die Kapseln fragil, das heißt, sie können eingeschlossenes Mittel aufgrund mechanischer Beanspruchung wie Reibung, Druck oder Scherbeanspruchung, welche die Hülle der Kapseln aufbricht, freigeben. In einer anderen Ausführungsform ist die Kapsel thermisch labil, das heißt, eingeschlossene Stoffe können freigesetzt werden, wenn die Kapseln einer Temperatur von mindestens 70°C, vorzugsweise von mindestens 60°C, bevorzugt dazu von mindestens 50°C und insbesondere von mindestens 40°C ausgesetzt wird.

In einer weiteren bevorzugten Ausführungsform kann die Kapsel für das/die eingeschlossenen Vorteilsmittel nach Einwirkung von Strahlung bestimmter Wellenlänge, vorzugsweise durch die Einwirkung von Sonnenlicht durchlässig werden.

Möglich ist zudem, dass die Kapseln fragil und gleichzeitig thermisch labil und/oder instabil gegenüber Strahlung bestimmter Wellenlänge sind.

Geeignete Mikrokapseln können wasserlöslich und/oder wasserunlöslich sein, bevorzugt handelt es sich aber um wasserunlösliche Kapseln. Die Wasserunlöslichkeit der Kapseln hat den Vorteil, dass diese Wasch-, Reinigungs- oder andere Behandlungsanwendungen überdauern können und so in der Lage sind, das mindestens eine Vorteilsmittel erst im Anschluss an den wässrigen Wasch-, Reinigungs- oder Behandlungsprozess abzugeben, wie beispielsweise beim Trocknen durch bloße Temperaturerhöhung oder durch Sonneneinstrahlung oder insbesondere bei Reibung der Oberfläche.

Besonders bevorzugt sind, in einigen Ausführungsformen, wasserunlösliche Kapseln, die durch Reibung aufgebrochen werden.

Der Begriff "aufreibbare" oder "durch Reibung aufbrechbare" Kapseln meint insbesondere solche Kapseln, welche, wenn sie an einer damit behandelten Oberfläche (z.B. textile Oberfläche) haften, durch mechanisches Reiben oder durch Druck geöffnet bzw. aufgebrochen werden können, so dass eine Inhaltsfreisetzung erst als Resultat einer mechanischen Einwirkung resultiert, z.B. wenn man sich mit einem Handtuch, auf welchem solche Kapseln abgelagert sind, die Hände abtrocknet.

Vorteilhaft einsetzbare, aufreibbare Kapseln können mittlere Durchmesser d₅₀ von < 250 µm, vorzugsweise im Bereich von 1 bis 100 µm auf, vorzugsweise zwischen 3 und 95 µm, insbesondere zwischen 4 und 90 µm, z.B. zwischen 5 und 80 µm, beispielsweise zwischen 5 und 40 µm aufweisen. Der d₅₀-Wert gibt dabei den Durchmesser an, der sich ergibt, wenn 50 Gew.-% der Kapseln einen geringeren Durchmesser und 50 Gew.-% der Kapseln einen größeren Durchmesser als der festgestellte d₅₀-Wert aufweisen. Es ist weiterhin bevorzugt, dass der d₉₀-Wert der Teilchengrößenverteilung der Mikrokapseln < 70 µm, bevorzugt < 60 µm, besonders bevorzugt < 50 m beträgt. Der d₉₀-Wert der Teilchengrößenverteilung ist der Wert, bei dem 90% aller Teilchen kleiner und 10% der Teilchen größer als dieser Wert sind.

Die den Kern bzw. (gefüllten) Hohlraum umschließende Schale der Kapseln hat vorzugsweise eine durchschnittliche Dicke im Bereich zwischen ungefähr rund 50 und 500 nm, vorzugsweise zwischen rund 100 nm und etwa 250 nm. Kapseln sind insbesondere dann gut aufreibbar, wenn sie innerhalb der zuvor angegebenen Bereiche betreffend den mittleren Durchmesser und betreffend die durchschnittliche Dicke liegen.

Der d₅₀-Wert gibt dabei den Durchmesser an, der sich ergibt, wenn 50 Gew.-% der Kapseln einen geringeren Durchmesser und 50 Gew.-% der Kapseln einen größeren Durchmesser als der festgestellte d₅₀-Wert aufweisen. Es ist weiterhin bevorzugt, dass der d₉₀-Wert der Teilchengrößenverteilung der Mikrokapseln < 70 µm, bevorzugt < 60 µm, besonders bevorzugt < 50 m beträgt. Der d₉₀-Wert der Teilchengrößenverteilung ist der Wert, bei dem 90 % aller Teilchen kleiner und 10% der Teilchen größer als dieser Wert sind.

Die Bestimmung der Durchmesser der Kapseln bzw. der Teilchengröße der Mikrokapseln kann über übliche Methoden erfolgen. Sie kann beispielsweise mit Hilfe dynamischer Lichtstreuung bestimmt werden, die üblicherweise an verdünnten Suspensionen, die z.B. 0,01 bis 1 Gew.-% Kapseln enthalten, durchgeführt werden kann. Sie kann auch durch die Auswertung lichtmikroskopischer oder elektronenmikroskopischer Aufnahmen von Kapseln erfolgen.

In verschiedenen Ausführungsformen weist eine erfindungsgemäße Mikrokapsel einen mittleren Durchmesser d₅₀ von ungefähr 1 bis 80 µm, vorzugsweise ungefähr 5 bis 40 µm, insbesondere ungefähr 20 bis 35 µm, beispielsweise ungefähr 22 bis ungefähr 33 µm, auf.

Das Wandmaterial der Mikrokapseln umfasst vorzugsweise die oben genannten Verbindungen, z.B. Polyurethane, Polyolefine, Polyamide, Polyester, Polysaccharide, Epoxidharze, Silikonharze und/oder Polykondensationsprodukte aus Carbonyl-Verbindungen und NH-Gruppen enthaltenden Verbindungen. Dies entspricht einer bevorzugten Ausführungsform der Erfindung. Bevorzugt können beispielsweise Melamin-Harnstoff-Formaldehyd-Mikrokapseln oder Melamin-Formaldehyd-Mikrokapseln oder Harnstoff-Formaldehyd-Mikrokapseln eingesetzt werden. Besonders bevorzugt sind Mikrokapseln auf Basis von Melamin-Formaldehyd-Harzen.

Das allgemeine Vorgehen bei der Mikrokapselherstellung als solches ist dem Fachmann seit langem wohlbekannt. Besonders geeignete Verfahren zur Mikrokapselherstellung sind prinzipiell z. B. in US 3,516,941, in US 3,415,758 oder auch in EP 0 026 914 A1 beschrieben. Letztgenannte beschreibt beispielsweise die Mikrokapselherstellung durch säureinduzierte Kondensation von Melamin-Formaldehyd-Vorkondensaten und/oder deren C₁₋₄-Alkylethern in Wasser, in dem das den Kapselkern bildende hydrophobe Material dispergiert ist, in Gegenwart eines Schutzkolloids.

Erfindungsgemäß enthalten die Mikrokapseln in ihrem Kern mindestens ein Geruchsneutralisierungsmittel, wie voranstehend definiert.

In verschiedenen Ausführungsformen umfasst der Kern der Mikrokapseln des Weiteren mindestens einen Kieselsäureester der allgemeinen Formel (III): 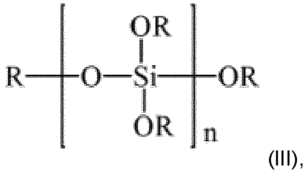 wobei, in den Verbindungen gemäß Formel (III), alle R jeweils unabhängig voneinander ausgewählt sind aus der Gruppe bestehend aus H, geradkettigen oder verzweigten, gesättigten oder ungesättigten, substituierten oder unsubstituierten C₁₋₆-Kohlenwasserstoffresten und Duftstoffalkoholresten; und n Werte aus dem Bereich 2 bis 100 annimmt.

In verschiedenen Ausführungsformen der Erfindung kann der Kern der Mikrokapsel auch die Verbindungen der Formel (III) enthalten ohne dass eine Verbindung der Formel (II) enthalten ist. In derartigen Ausführungsformen gelten die oben für die Verbindungen der Formel (II) gemachten Angaben auch für solche der Formel (III).

In den Verbindungen der allgemeinen Formel (III) liegen Duftstoff-Alkohole vor, die mit Kieselsäuren und deren Derivaten umgesetzt sind.

Oligokieselsäureester niederer Alkohole sind kommerziell erhältlich, wobei üblicherweise Methanol, Ethanol, n-Propanol, iso-Propanol, n-Butanol, iso-Butanol und tert.-Butanol zur Veresterung einge-setzt wurden. Die Darstellung nicht vollständig mit Duftstoffalkoholen umgeesterter Oligokieselsäu-reester führt zu Kieselsäureester-Mischungen in denen ein Teil der Reste R ausgewählt ist aus der Gruppe Methyl, Ethyl, n-Propyl, iso-Propyl, n-Butyl, iso-Butyl und tert.-Butyl. Solche Verbindungen sind im Rahmen der vorliegenden Erfindung bevorzugt.

In verschiedenen Ausführungsformen ist ein Teil der Reste R, vorzugsweise mindestens 5 mol-% der Reste R, ausgewählt aus der Gruppe bestehend aus Methyl, Ethyl, n-Propyl, iso-Propyl, n-Butyl, iso-Butyl und tert-Butyl.

Erfindungsgemäß sind solche Oligokieselsäureester, die mindestens einen Rest R aus der Gruppe der Duftstoff-Alkohol-Reste enthalten, bevorzugt.

Die Herstellung der genannten Verbindungen gelingt durch einfache Umesterung von Oligokieselsäureestern niederer Alkohole mit Duftstoffalkoholen, wobei sowohl einzelne Duftstoffalkohole als auch Duftstoffalkoholgemische eingesetzt werden können. Je nach Reaktionszeit und -bedingungen werden die niederen Alkohole abgespalten und die Duftstoff- bzw. Biozidalkohole gebunden, wobei die Alkohole entlang von Si-O-Si-Ketten oder -Ringen leichter ausgetauscht werden als die terminalen Alkohole. Üblicherweise werden als Edukte die handelsüblichen Kieselsäureester eingesetzt. Hier ist insbesondere der Ethanol-Ester zu nennen. Die Umesterung kann dabei ausschließlich durch Temperaturerhöhung und Abdestillation der leichtflüchtigen Nebenprodukte gesteuert werden. bevorzugt ist es jedoch, wenn zur Umesterung Katalysatoren eingesetzt werden. Es handelt sich dabei üblicherweise um Lewis-Säuren, vorzugsweise um Aluminiumtetraisopropylat, Titantetraisopropylat, Siliciumtetrachlorid oder basische Katalysatoren oder auch um Zubereitungen wie beispielsweise aus Aluminiumoxid mit Kaliumfluorid. Die so gebildeten oligomeren Kieselsäureester weisen dann zumindest teilweise Duftstoffalkoholreste auf. Üblicherweise enthalten die resultierenden Ester jedoch auch noch Reste niederer Alkohole. Soweit bei der Herstellung der Kieselsäureester geringe Mengen Wasser oder anderer Wasserstoff-acider Verbindungen anwesend sind, findet auch Austausch von Alkohol-Resten gegen OH-Gruppen statt. Dementsprechend enthalten die erfindungsgemäßen Kieselsäureester-Mischungen üblicherweise als Rest R teilweise auch Wasserstoff.

Die vollständig umgeesterten Oligokieselsäureester sind im Rahmen der vorliegenden Erfindung besonders bevorzugt. Insbesondere ist es dabei bevorzugt, wenn diese Ester nur einen einzigen Rest R enthalten, d.h. nur einen einzigen Duftstoffalkohol enthalten.

Die Oligomerisierungsgrade "n" der erfindungsgemäßen Kieselsäureester liegen zwischen 2 und 100, vorzugsweise zwischen 2 bis 50, bevorzugt zwischen 2 und 20. In bevorzugten Verbindungen nimmt n Werte zwischen 2 und 15, vorzugsweise zwischen 2 und 12 und insbesondere zwischen 3 und 10 unter besonderer Bevorzugung der Werte 4, 5, 6, 7 und 8, an.

Unter dem Begriff Duftstoff-Alkohole werden im Rahmen der vorliegenden Erfindung Duftstoffe verstanden, die über freie Hydroxylgruppen verfügen, welche veresterbar sind, unabhängig davon, wie das Molekül weiter aufgebaut ist. So lassen sich auch Salicylsäureester als Duftstoffalkohole einsetzen. Aus der großen Gruppe der Duftstoffalkohole seien, im Kontext der vorliegenden Erfindung, beispielhaft und ohne Einschränkung, 10-Undecen-1-ol, 2,6-Dimethylheptan-2-ol, 2-Methyl-butanol, 2-Methylpentanol, 2- Phenoxyethanol, 2-Phenylpropanol, 2-tert.-Butycyclohexanol, 3,5,5-Trimethylcyclohexanol, 3-Hexanol, 3-Methyl-5-phenyl-pentanol, 3-Octanol, 3-Phenyl-propanol, 4-Heptenol, 4-Isopropylcyclohexanol, 4-tert.-Butycyclohexanol, 6,8-Dimethyl-2-nona-nol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol und Zimtalkohol genannt. Im Rahmen der vorliegenden Erfindung sind Kieselsäureester bevorzugt, in denen jedes R unabhängig voneinander ausgewählt ist aus der Gruppe der Reste der vorstehend aufgezählten Duftstoffalkohole sowie Mischungen daraus. Weitere geeignete Biozidalkohole sind 1,2-Propylenglykol, Glycerin, Citronensäure und deren Ester, Milchsäure und deren Ester, Salicylsäure und deren Ester, 2-Benzyl-4-chlorphenol und 2,2'-Methylen-bis-(6-brom-4-chlorphenol).

In verschiedenen Ausführungsformen sind mindestens 10 mol-%, vorzugsweise mindestens 20 mol-% und insbesondere bevorzugt sogar mehr als 40 mol-% der Reste R ausgewählt aus der Gruppe bestehend aus den Resten der Duftstoffalkohole 10-Undecen-1-ol, 2,6- Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl5-phenyl-pentanol, 3-Octanol, 3-Phenyl-propanol, 4-Heptenol, 4-Isopropylcyclohexanol, 4-tert.-Butycyclohexanol, 6,8-Dimethyl-2-nona-nol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol, Zimtalkohol und Mischungen daraus.

Bevorzugt werden Phenylethylkieselsäureester, Geranylkieselsäureester, Citronellylkieselsäureester, Cynamylkieselsäureester, Hexenylkieselsäureester, Nonadienylkieselsäureester, Octenylkieselsäureester oder Mischungen aus zwei oder mehr dieser Kieselsäureester eingesetzt.

In verschiedenen Ausführungsformen kann der Kern weitere Vorteilsmittel enthalten. In einigen Ausführungsformen handelt es sich bei einem solchen weiteren Vorteilsmittel um ein Parfümöl. Es kann allerdings bevorzugt sein, dass der Kern kein weiteres solches Vorteilsmittel oder Parfümöl enthält. Derartige Ausführungsformen in denen der Kern frei von weiterem Parfümöl ist erfassen allerdings solche Mikrokapseln, in deren Kern freies Parfümöl als (spontanes) Freisetzungsprodukt aus Geruchsneutralisierungsmitteln der Erfindung vorliegt. Diese werden in einem derartigen Fall aber nicht bewusst in freier Form zugeführt, sondern stammen aus dem eingesetzten Geruchsneutralisierungsmittel. Diese können allerdings auch in freier oder in separater verkapselter Form in dem Mittel enthalten sein.

Als Parfümöle können alle dafür bekannten Stoffe und Gemische eingesetzt werden. Im Sinne dieser Erfindung werden die Begriffe "Riechstoff(e)", "Duftstoffe" und "Parfümöl(e)" synonym gebraucht. Damit sind insbesondere all jene Stoffe oder deren Gemische gemeint, die von Mensch und Tier als Geruch empfunden werden, insbesondere vom Mensch als Wohlgeruch empfunden werden.

Als Duftkomponenten können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein.

Duftstoffverbindungen vom Typ der Aldehyde sowie vom Typ der Ketone wurden bereits vorangehend aufgelistet.

Duftstoffverbindungen vom Typ der Alkohole wurden ebenfalls bereits vorangehend exemplarisch aufgelistet.

Duftstoffverbindungen vom Typ der Ester sind z.B. Benzylacetat, Phenoxyethylisobutyrat, p-tert-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat.

Zu den Ethern zählen beispielsweise Benzylethylether und Ambroxan. Zu den Kohlenwasserstoffen gehören hauptsächlich Terpene wie Limonen und Pinen.

Bevorzugt werden Mischungen verschiedener Duftstoffe verwendet, die gemeinsam eine ansprechende Duftnote erzeugen. Ein derartiges Gemisch an Duftstoffen kann auch als Parfüm oder Parfümöl bezeichnet werden. Solche Parfümöle können auch natürliche Duftstoffgemische enthalten, wie sie aus pflanzlichen Quellen zugänglich sind.

Zu den Duftstoffen pflanzlichen Ursprungs zählen ätherische Öle wie Angelikawurzelöl, Anisöl, Arnikablütenöl, Basilikumöl, Bayöl, Champacablütenöl, Citrusöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, jasminöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Labdanumöl, Lavendelöl, Lemongrasöl, Lindenblütenöl, Limettenöl, Mandarinenöl, Melissenöl, Minzöl, Moschuskörneröl, Muskatelleröl, Myrrhenöl, Nelkenöl, Neroliöl, Niaouliöl, Olibanumöl, Orangenblütenöl, Orangenschalenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Salbeiöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphthol-methylether, Nerol, n-Nonylaldehyd, Nonylalkohol, n-Octylaldehyd, p-Oxy-Acetophenon, Pentadekanolid, beta-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, gamma-Undelacton, Vanillin, Veratrumaldehyd, Zimtaldehyd, Zimtalkohol, Zimtsäure, Zimtsäureethylester, Zimtsäurebenzylester, Diphenyloxid, Limonen, Linalool, Linalylacetat und - Propionat, Melusat, Menthol, Menthon, Methyl-n-heptenon, Pinen, Phenylacetaldehyd, Terpinylacetat, Citral, Citronellal, sowie Mischungen daraus.

Ebenfalls können Gemische der genannten Stoffe verwendet werden.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Analog zu der Beschreibung in der internationalen Patentveröffentlichung WO 2016/200761 A2 können Kopf-, Herz- und Basisnote anhand ihres Dampfdrucks (mittels der in der WO 2016/200761 beschriebenen Testverfahren bestimmbar) wie nachstehend klassifiziert werden:
Kopfnote: Dampfdruck bei 25°C: >0,0133 kPa
Herznote: Dampfdruck bei 25°C: 0,0133 bis 0,000133 kPa
Basisnote: Dampfdruck bei 25°C: <0,000133 kPa

Zu den haftfesten Riechstoffe, die im Rahmen der vorliegenden Erfindung einsetzbar sind, gehören beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopalvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Höhersiedende bzw. festen Riechstoffe natürlichen oder synthetischen Ursprungs umfassen beispielsweise: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecyl-aldehyd, Eugenol, Eugenolmethylether, Eukalyptol, Farnesol, Fenchon, Fenchylacetat, Geranyl-acetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenol-methylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylaceto-phenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenyl-ethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Bevorzugt einsetzbare Riechstoffverbindungen vom Typ der Aldehyde sind Hydroxycitronellal (CAS 107-75-5), Helional (CAS 1205-17-0), Citral (5392-40-5), Bourgeonal (18127-01-0), Triplal (CAS 27939-60-2), Ligustral (CAS 68039-48-5), Vertocitral (CAS 68039-49-6), Florhydral (CAS 125109-85-5), Citronellal (CAS 106-23-0), Citronellyloxyacetaldehyd (CAS 7492-67-3).

Zusätzlich oder alternativ zu den vorstehend genannten Riechstoffen können auch die in der WO 2016/200761 A2 beschriebenen Riechstoffe, insbesondere die in den Tabellen 1, 2 und 3 genannten Riechstoffe, sowie die in den Tabellen 4a und 4b aufgelisteten Modulatoren eingesetzt werden. Diese Veröffentlichung ist hierin durch Bezugnahme in ihrer Gesamtheit eingeschlossen.

Geeignete Parfümöle als Bestandteil der erfindungsgemäßen Zusammensetzung können auch in Form einer Parfümölzubereitung enthalten und beispielsweise mindestens einen weiteren Aktivstoff in Ölform umfassen. Geeignete Aktivstoffe in Ölform sind in diesem Zusammenhang solche, welche für Wasch-, Reinigungs-, Pflege- und/oder Veredelungszwecke geeignet sind, insbesondere
(a) Textilpflegestoffe, wie vorzugsweise Silikonöle, und/oder
(b) Hautpflegestoffe, wie vorzugsweise Vitamin E, natürliche Öle und/oder kosmetische Öle.

Hautpflegende Aktivstoffe sind all jene Aktivstoffe, die der Haut einen sensorischen und/oder kosmetischen Vorteil verleihen. Hautpflegende Aktivstoffe sind bevorzugt ausgewählt aus den nachfolgenden Substanzen:
a) Wachse wie beispielsweise Carnauba, Spermaceti, Bienenwachs, Lanolin und/oder Derivate derselben und andere.
b) Hydrophobe Pflanzenextrakte
c) Kohlenwasserstoffe wie beispielsweise Squalene und/oder Squalane
d) Höhere Fettsäuren, vorzugsweise solche mit wenigstens 12 Kohlenstoffatomen, beispielsweise Laurinsäure, Stearinsäure, Behensäure, Myristinsäure, Palmitinsäure, Ölsäure, Linolsäure, Linolensäure, Isostearinsäure und/oder mehrfach ungesättigte Fettsäuren und andere.
e) Höhere Fettalkohole, vorzugsweise solche mit wenigstens 12 Kohlenstoffatomen, beispielsweise Laurylalkohol, Cetylalkohol, Stearylalkohol, Oleylalkohol, Behenylalkohol, Cholesterol und/oder 2-Hexadecanaol und andere.
f) Ester, vorzugsweise solche wie Cetyloctanoate, Lauryllactate, Myristyllactate, Cetyllactate, Isopropylmyristate, Myristylmyristate, Isopropylpalmitate, Isopropyladipate, Butylstearate, Decyloleate, Cholesterolisostearate, Glycerolmonostearate, Glyceroldistearate, Glyceroltristearate, Alkyllactate, Alkylcitrate und/oder Alkyltartrate und andere.
g) Lipide wie beispielsweise Cholesterol, Ceramide und/oder Saccharoseester und andere.
h) Vitamine wie beispielsweise die Vitamine A, C und E, Vitaminalkylester, einschließlich Vitamin-C-Alkylester und andere.
i) Sonnenschutzmittel
j) Phospholipide
k) Derivate von alpha-Hydroxysäuren
l) Germizide für den kosmetischen Gebrauch, sowohl synthetische wie beispielsweise Salicylsäure und/oder andere als auch natürliche wie beispielsweise Neemöl und/oder andere.
m) Silikone
n) Natürliche Öle, z.B. Mandelöl
sowie Mischungen jeglicher vorgenannter Komponenten.

In verschiedenen Ausführungsformen kann der Kern der Mikrokapseln, neben dem mindestens einen Geruchsneutralisierungsmittel und ggf. mindestens einen Kieselsäureesterverbindung des Weiteren insbesondere mindestens ein Duftstoffaldehyd und/oder Duftstoffketon umfassen, jeweils vorzugsweise ausgewählt aus der Gruppe der vorstehend genannten Duftstoffaldehyde und Duftstoffketone. Es kann aber auch bevorzugt sein, dass der Kern der Mikrokapseln frei von derartigen zusätzlichen Duftstoffen ist und diese separat in das Mittel formuliert werden.

In bevorzugten Ausführungsformen ist die Mikrokapsel, insbesondere der Kern der Mikrokapsel, neben dem mindestens einen Geruchsneutralisierungsmittel, d.h. mindestens einer 1-Aza-3,7-dioxabicyclo[3.3.0]octan-Verbindung der allgemeinen Formel (II) und ggf. mindestens einer Kieselsäureesterverbindung der Formel (III) und/oder ggf. mindestens einer Verbindung der Formel (IV), frei von zusätzlichen freien Duftstoffen, d.h. die Mikrokapsel, insbesondere der Kern der Mikrokapsel, enthält keine zusätzlichen freien Duftstoffe/Riechstoffe wie oben definiert.

"Zusätzliche freie Duftstoffe" im Rahmen der vorliegenden Erfindung beziehen sich auf weitere freie Duftstoffe, die zusätzlich zu den Verbindungen der Formel (II) und ggf. den Verbindungen der Formeln (III) und/oder (IV) in den Mikrokapseln, insbesondere im Kern der Mikrokapseln, enthalten sein können. Duftstoffaldehyde, Duftstoffketone und/oder Duftstoffalkohole, die aus den Verbindungen der Formeln (II), (III) und/oder (IV) freigesetzt werden können, sind hiervon nicht umfasst. Gemäß der vorliegenden Erfindung sind die Mikrokapseln, insbesondere die Kerne der Mikrokapseln, frei von zusätzlichen freien Duftstoffen neben den Verbindungen der Formel (II) und ggf. den Verbindungen der Formeln (III) und/oder (IV), abgesehen von den freien Duftstoffaldehyden, Duftstoffketonen und/oder Duftstoffalkoholen, die aus den Verbindungen der Formeln (II), (III) und/oder (IV) freigesetzt werden können.

In verschiedenen Ausführungsformen können geeignete Mikrokapseln des Weiteren Pflanzenextrakte als Aktivstoff enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel geeignet. Insbesondere bevorzugt sind Extrakte aus Aloe Vera.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2-80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, als Aktivstoff mehrere, insbesondere zwei verschiedene Pflanzenextrakte einzusetzen.

In verschiedenen Ausführungsformen enthalten die voranstehend beschriebenen Mikrokapseln entsprechend mindestens ein Geruchsneutralisierungsmittel sowie ggf. mindestens einen Kieselsäureester der allgemeinen Formel (III), welche auch Bestandteil einer Vorteilsmittelzusammensetzung sein können, also in Kombination mit weiteren Duft- oder Riechstoffen, beispielsweise Parfümölen, wie voranstehend beschrieben, enthalten sein können. In einigen Ausführungsformen ist das mindestens eine Geruchsneutralisierungsmittel sowie ggf. weitere Vorteilsmittel und/oder mindestens ein Kieselsäureester, wie voranstehend definiert, in einer Menge von ungefähr 30 bis ungefähr 95 Gew.-%, vorzugsweise mindestens 40 bis ungefähr 90 Gew.-%, noch bevorzugter mindestens 50 bis ungefähr 90 Gew.-%, insbesondere mindestens 60 bis ungefähr 90 Gew.-%, jeweils bezogen auf das Gesamtgewicht der Mikrokapseln, in diesen enthalten.

Gemäß der vorliegenden Erfindung können geeignete Mikrokapseln auch in Form eines (Kapsel-)Slurries, d.h. einer Aufschlämmung der Kapseln in einem flüssigen Medium, in der erfindungsgemäßen Zusammensetzung enthalten sein.

Der Begriff "Slurry" bezeichnet im Kontext der vorliegenden Erfindung eine, typischerweise wässrige, Aufschlämmung der Mikrokapseln, wie voranstehend definiert. Das flüssige Medium besteht vorzugsweise zum überwiegenden Anteil, d.h. zu mehr als 50 Gew.-% aus Wasser, kann aber auch vollständig, d.h. zu 100 % aus Wasser bestehen. Der Slurry ist vorzugsweise gießbar, d.h. er lässt sich aus einem Gefäß durch Neigen des Gefäßes ausgießen. Unter einem gießbaren Slurry wird insbesondere ein Kapsel-Flüssigkeitsgemisch verstanden, welches insbesondere bei der Verarbeitungstemperatur, vorzugsweise bei maximal 40 °C, insbesondere bei maximal 20 °C eine Viskosität unterhalb von 10-10⁴ mPas (Brookfield-Rotationsviskosimeter; Spindel 2, 20 U/min.) aufweist.

Der Slurry kann weitere Hilfsstoffe enthalten, beispielsweise solche, die eine bestimmte Haltbarkeit oder Stabilität sicherstellen. Häufig verwendete Hilfsstoffe schließen beispielsweise Tenside, insbesondere anionische und/oder nichtionische Tenside ein. Entsprechende Kapselslurries sind kommerziell erhältlich und dem Fachmann als solches bekannt.

In verschiedenen Ausführungsformen sind die vorangehend beschriebenen Kapseln in einer Menge von 5 bis 80 Gew.-%, vorzugsweise 10 bis 70 Gew.-%, insbesondere 20 bis 60 Gew.-%, beispielsweise 30-40 Gew.-% in dem Slurry enthalten.

In verschiedenen Ausführungsformen sind die voranstehend beschriebenen Mikrokapseln in einer Menge von ungefähr 0,01 bis 50 Gew.-%, vorzugsweise ungefähr 0,1 bis 25 Gew.-%, insbesondere 0,1 bis 10 Gew.-%, jeweils bezogen auf das Gesamtgewicht der erfindungsgemäßen Zusammensetzung, in dieser enthalten.

In verschiedenen Ausführungsformen sind die voranstehend beschriebenen Mikrokapseln insbesondere in einer Menge von ungefähr 0,01 bis 50 Gew.-%, vorzugsweise ungefähr 0,1 bis 25 Gew.-%, insbesondere 0,1 bis 10 Gew.-%, jeweils bezogen auf das Gesamtgewicht der erfindungsgemäßen Zusammensetzung, in dieser enthalten, wobei es sich bei der Zusammensetzung um eine Weichspüler-Zusammensetzung oder Fabric Finisher-Zusammensetzung handelt. Bevorzugt liegen die Mikrokapseln, in dieser Ausführungsform, als Kapsel-Slurry vor.

In verschiedenen Ausführungsformen sind die voranstehend beschriebenen Mikrokapseln insbesondere in einer Menge von ungefähr 0,01 bis 50 Gew.-%, vorzugsweise ungefähr 0,1 bis 25 Gew.-%, insbesondere 0,1 bis 10 Gew.-%, jeweils bezogen auf das Gesamtgewicht der erfindungsgemäßen Zusammensetzung, in dieser enthalten, wobei es sich bei der Zusammensetzung um eine Flüssigwaschmittel-Zusammensetzung handelt. Bevorzugt liegen die Mikrokapseln, in dieser Ausführungsform, als Kapsel-Slurry vor.

In verschiedenen Ausführungsformen ist das mindestens eine Geruchsneutralisierungsmittel in einer Menge von ungefähr 0,1 bis ungefähr 40 Gew.-%, vorzugsweise ungefähr 0,1 bis ungefähr 20 Gew.-%, jeweils bezogen auf das Gesamtgewicht der Zusammensetzung, in dieser enthalten.

In verschiedenen Ausführungsformen ist der mindestens eine Kieselsäureester, wie voranstehend definiert, in einer Menge von ungefähr 0,1 bis ungefähr 40 Gew.-%, vorzugsweise ungefähr 0,1 bis ungefähr 20 Gew.-%, jeweils bezogen auf das Gesamtgewicht der Zusammensetzung, in dieser enthalten.

In verschiedenen Ausführungsformen umfasst die erfindungsgemäße Zusammensetzung des Weiteren mindestens ein weiteres Vorteilsmittel, d.h., zusätzlich zu den vorgenannten Vorteilsmitteln, beispielsweise Parfümölen, welche in verkapselter Form vorliegen, mindestens ein weiteres, unverkapseltes Vorteilsmittel. Geeignete weitere Vorteilsmittel unterliegen erfindungsgemäß keinerlei Beschränkungen und können ausgewählt sein aus im Stand der Technik bekannten, zur Verwendung in Wasch-, Reinigungs- und Behandlungsverfahren geeigneten Vorteilsmitteln, wie beispielsweise Duft- und Riechstoffen, Duftstoffvorläuferverbindungen, hautpflegenden Aktivstoffen, usw.

In verschiedenen Ausführungsformen handelt es sich bei der erfindungsgemäßen Zusammensetzung, wie vorangehend beschrieben, um ein Wasch-, Reinigungs- oder Behandlungsmitteladditiv. Ein solches Additiv kann beispielsweise, ohne Einschränkung, in Form einer flüssigen oder festförmigen Zusammensetzung vorliegen.

"Festförmig", wie hierin verwendet, bezeichnet eine Pulver-, Granulat-, Extrudat- oder Kompakt-Zusammensetzung.

"Flüssig", wie im Kontext der vorliegenden Erfindung verwendet, bezeichnet alle fließfähigen Zusammensetzungen (bei 20 °C, 1,013 bar), einschließlich Gelen und Pasten-artigen Zusammensetzungen, sowie des Weiteren Nicht-Newtonschen Flüssigkeiten, die eine Fließgrenze aufweisen.

Ein weiterer Gegenstand der vorliegenden Erfindung ist ein Mittel umfassend eine wie hierin beschriebene Zusammensetzung, bei welcher es sich gemäß einiger Ausführungsformen um ein Wasch-, Reinigungs- oder Behandlungsmitteladditiv handeln kann. Entsprechend handelt es sich, in einigen Ausführungsformen, bei dem erfindungsgemäßen Mittel um ein Wasch-, Reinigungs- oder Behandlungsmittel, beispielsweise ein Kosmetikprodukt, Wasch-, Reinigungs- oder Textilbehand lungsmittel.

Erfindungsgemäße Mittel eignen sich zum Waschen, Reinigen, Konditionieren, Pflegen und/oder Färben von harten oder weichen Oberflächen. Im Sinne dieser Anmeldung kann es sich bei erfindungsgemäßen Mitteln folglich um Wasch-, Reinigungs-, Nachbehandlungs- und/oder kosmetische Mittel handeln. Erfindungsgenmäße Mittel schließen auch Mittel für den Air-Care-Bereich mit ein, sind also geeignet für die Raumluftverbesserung bzw. Fehlgeruchsbekämpfung.

Harte Oberflächen im Sinne dieser Anmeldung sind dabei Fenster, Spiegel und weitere Glasoberflächen, Oberflächen aus Keramik, Kunststoff, Metall oder auch Holz sowie lackiertes Holz, die sich in Haushalt und Gewerbe finden, etwa Badkeramik, Koch- und Speisegeschirr, Küchenoberflächen oder Fußböden. Weiche Oberflächen im Sinne dieser Anmeldung sind textile Flächengebilde, Haut sowie Haare.

Mittel zum Waschen von harten oder weichen Oberflächen im Sinne dieser Anmeldung sind Textilwaschmittel, z.B. in Form von Pulvern, Granulaten, Perlen, Tabletten, Pasten, Gelen, Tüchern, Stücken oder Flüssigkeiten vorliegenden Formulierungen.

Mittel zum Reinigen von harten oder weichen Oberflächen im Sinne dieser Anmeldung umfassen alle Reiniger für harte oder weiche Oberflächen, insbesondere Geschirrspülmittel, Allzweckreiniger, WC-Reiniger, Sanitärreiniger sowie Glasreiniger, Zahncremes, Hautwaschmittel, wie Duschgele, oder Haarwaschmittel.

Mittel zum Konditionieren von harten oder weichen Oberflächen im Sinne dieser Anmeldung sind Weichspüler, Duftspüler, Konditioniertücher für die Anwendung im Wäschetrockner, Hygienespüler, Deodorantien, Antitranspirantien, Haarkonditioniermittel, Stylingmittel und/oder Haarfestigungsmittel.

Mittel zur Pflege von harten oder weichen Oberflächen im Sinne dieser Anmeldung sind Textilpflegemittel, Haarpflegemittel oder Hautbehandlungsmittel, wie beispielsweise Cremes, Lotionen oder Gele.

Mittel zum Färben von harten oder weichen Oberflächen im Sinne dieser Anmeldung sind Haarfärbe- und Haartönungsmittel und Mittel zum Aufhellen keratinischer Fasern.

Mittel für den Air-Care-Bereich, mit anderen Worten Mittel zur Behandlung (Verbesserung) der Raumluft, schließen ein, sind aber nicht beschränkt auf, Raumsprays, Textilsprays und Duftabgabesysteme.

In einer bevorzugten Ausführungsform handelt es sich bei der Oberfläche um eine textile Oberfläche. Wenn es sich bei der Oberfläche um eine textile Oberfläche handelt ist insbesondere bevorzugt, wenn das Mittel zum Waschen, Reinigen, Konditionieren, Pflegen und/oder Färben von harten oder weichen Oberflächen ein Wasch-, Reinigungs- oder Nachbehandlungsmittel ist.

In einer weiteren Ausführungsform handelt es sich bei der Oberfläche um eine Körperstelle, insbesondere um Haut und/oder Haare. Wenn es sich bei der Oberfläche um eine Körperstelle, insbesondere um Haut und/oder Haare handelt, ist bevorzugt, dass das Mittel zum Waschen, Reinigen, Konditionieren, Pflegen und/oder Färben von harten oder weichen Oberflächen eine kosmetische Zusammensetzung ist.

Ein erfindungsgemäßes Mittel umfasst mindestens eine Art einer wie hierin definierten und beschriebenen Zusammensetzung. In bevorzugten Ausführungsformen enthält ein solches Mittel mindestens eine erfindungsgemäße Zusammensetzung in einer Menge von bis zu ungefähr 30 Gew.-%, vorzugsweise bis zu ungefähr 20 Gew.-%, noch bevorzugter bis zu ungefähr 15 Gew.-%, insbesondere bis zu ungefähr 10 Gew.-%, beispielsweise bis zu 1, 2, 3, 4, 5, 6, 7, 8, 9 oder 10 Gew.-%.

Neben der hierin beschriebenen Zusammensetzung kann ein erfindungsgemäßes Mittel darüber hinaus übliche und dem Fachmann als solche bekannte weitere Inhaltsstoffe, beispielsweise mindestens einen oder vorzugsweise mehrere Stoffe aus der Gruppe der Enzyme, Tenside, Bleichmittel, Komplexbildner, Gerüststoffe, Elektrolyte, nichtwässrigen Lösungsmittel, pH-Stellmittel, weiteren Duftstoffe, weiteren Duftstoffträger, Fluoreszenzmittel, Farbstoffe, Hydrotrope, Schauminhibitoren, Silikonöle, Antiredepositionsmittel, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Bittermittel, Bügelhilfsmittel, Phobier- und Imprägniermittel, Quell- und Schiebefestmittel, weichmachenden Komponenten sowie UV-Absorber enthalten.

Weitere Gegenstände der vorliegenden Erfindung sind darüber hinaus die Verwendung einer erfindungsgemäßen Zusammensetzung oder eines erfindungsgemäßen Mittels zur Verminderung von Fehlgerüchen, sowie ein Verfahren zur Behandlung einer Oberfläche, zur Behandlung der Raumluft, oder zum Waschen und/oder Pflegen von Textilien, dadurch gekennzeichnet, dass in mindestens einem Verfahrensschritt eine erfindungsgemäße Zusammensetzung oder ein erfindungsgemäßes Mittel angewendet wird, wie voranstehend beschrieben.

Alle Sachverhalte, Gegenstände und Ausführungsformen, die für die erfindungsgemäßen Zusammensetzungen beschrieben sind, sind auch auf die vorstehend genannten Verfahren, Verwendungen und diese Zusammensetzungen enthaltenden Mittel anwendbar. Daher wird an dieser Stelle ausdrücklich auf die Offenbarung an entsprechender Stelle verwiesen mit dem Hinweis, dass diese Offenbarung auch für die vorstehenden beschriebenen Verfahren und Verwendungen gilt.

Die Erfindung wird im Folgenden anhand von Beispielen illustriert, ist aber nicht auf diese beschränkt.

### Beispiele

Im Folgenden aufgeführt sind beispielhafte Rezepturen gemäß der vorliegenden Erfindung sowie Vergleichsformulierungen. Entsprechende Formulierungen wurden in anwendungstechnischen Versuchsreihen auf ihre geruchliche Qualität (d.h. Intensität der Fehlgeruchsneutralisierung) hin überprüft. Die entsprechenden Ergebnisse sind tabellarisch zusammengefasst (Skala 1-10, wobei 1 sehr schwach und 10 sehr stark bedeutet).

### Beispiel 1

| | |
|---|---|
| Produkt: | Universalwaschmittel |
| Dosierung Produkt: | 50 ml |
| Waschbedingungen: | Hauptwaschgang bei 40 °C |
| Fehlgeruch: | Schweiß |
| Textilart: | Polyester |

**Tabelle 1**

| **Formulierung** | **Intensität des Fehlgeruchs** | | | | | |
|---|---|---|---|---|---|---|
| | nach | nach | nach | nach | nach | nach |
| | 0h | 2h | 4h | 6h | 24h | 48h |
| Unparfümierte Formulierung | 10 | 9 | 7 | 6 | 5 | 3 |
| 1 % Parfümierung enthaltend 5 % 1H,3H,5H-Oxazolo[3,4-c]oxazole, 3,5-diheptyldihydro-7a-methyl- - **nicht verkapselt** | 9 | 6 | 5 | 4 | 4 | 3 |
| 1 % Parfümierung enthaltend 5 % 1H,3H,5H-Oxazolo[3,4-c]oxazole, 3,5-diheptyldihydro-7a-methyl- **-verkapselt** | 7 | 4 | 3 | 2 | 2 | 2 |

### Beispiel 2

| | |
|---|---|
| Produkt: | Weichspüler |
| Dosierung Produkt: | 30 ml |
| Waschbedingungen: | Hauptwaschgang bei 40 °C |
| Fehlgeruch: | Schweiß |
| Textilart: | Baumwolle |

### Tabelle 2

| **Formulierung** | **Intensität des Fehlgeruchs** | | | | | |
|---|---|---|---|---|---|---|
| | nach | nach | nach | nach | nach | nach |
| | 0h | 2h | 4h | 6h | 24h | 48h |
| Unparfümierte Formulierung | 9 | 8 | 6 | 5 | 4 | 3 |
| 1,5 % Parfümierung enthaltend 5 % 1H,3H,5H-Oxazolo[3,4-c]oxazole, 3,5-diheptyldihydro-7a-methyl- - **nicht verkapselt** | 8 | 7 | 5 | 3,5 | 3 | 2,5 |
| 1,5 % Parfümierung enthaltend 5 % 1H,3H,5H-Oxazolo[3,4-c]oxazole, 3,5-diheptyldihydro-7a-methyl- **-verkapselt** | 6 | 4 | 2 | 2 | 1,5 | 1,5 |

### Beispiel 3

| | |
|---|---|
| Produkt: | Farbfangtuch |
| Dosierung Produkt: | 1 Tuch |
| Waschbedingungen: | Hauptwaschgang bei 40 °C |
| Fehlgeruch: | Schweiß |
| Textilart: | Baumwolle |

**Tabelle 3**

| **Formulierung** | **Intensität des Fehlgeruchs** | | | | | |
|---|---|---|---|---|---|---|
| | nach | nach | nach | nach | nach | nach |
| | 0h | 2h | 4h | 6h | 24h | 48h |
| Unparfümierte Formulierung | 10 | 9 | 9 | 8 | 7 | 5 |
| 4 % Parfümierung enthaltend 5 % 1H,3H,5H-Oxazolo[3,4-c]oxazole, 3,5-diheptyldihydro-7a-methyl- - **nicht verkapselt** | 8 | 7 | 5 | 4 | 3,5 | 3 |
| 4 % Parfümierung enthaltend 5 % 1H,3H,5H-Oxazolo[3,4-c]oxazole, 3,5-diheptyldihydro-7a-methyl- **-verkapselt** | 5 | 4 | 3 | 3 | 2 | 1,5 |

### Beispiel 4

| | |
|---|---|
| Produkt: | Universalwaschmittel |
| Dosierung Produkt: | 50 ml |
| Waschbedingungen: | Hauptwaschgang bei 40 °C |
| Fehlgeruch: | Moder |
| Textilart: | Polyester |

**Tabelle 4**

| **Formulierung** | **Intensität des Fehlgeruchs** | | | | | |
|---|---|---|---|---|---|---|
| | nach 0h | nach 2h | nach 4h | nach 6h | nach 24h | nach 48h |
| Unparfümierte Formulierung | 10 | 9 | 8 | 8 | 5 | 3 |
| 1 % Parfümierung enthaltend 5 % 1H,3H,5H-Oxazolo[3,4-c]oxazole, 3,5-diheptyldihydro-7a-methyl- **nicht verkapselt** | 8 | 6 | 4 | 4 | 3 | 3 |
| 1 % Parfümierung enthaltend 5 % 1H,3H,5H-Oxazolo[3,4-c]oxazole, 3,5-diheptyldihydro-7a-methyl- **-verkapselt** | 7 | 6 | 3 | 3 | 2 | 2 |

### Beispiel 5

| | |
|---|---|
| Produkt: | Fabric finisher |
| Dosierung Produkt: | 30 g |
| Waschbedingungen: | Hauptwaschgang bei 40 °C |
| Fehlgeruch: | Schweiß |
| Textilart: | Baumwolle |

**Tabelle 5**

| **Formulierung** | **Intensität des Fehlgeruchs** | | | | |
|---|---|---|---|---|---|
| | Dosierung | nach 0h (direkt nach der Anwendung des Fehlgeruchs) | nach 2h | nach 4h | nach 4h nach Reiben |
| Geruchsneutralisierer 1 - **nicht verkapselt** | 0,1 % | 7 | 6,0 | 5,0 | 4,0 |
| Geruchsneutralisierer 2 - **nicht verkapselt** | 0,1 % | 8 | 7,0 | 5,5 | 4,0 |
| Geruchsneutralisierer 3 - **nicht verkapselt** | 0,1 % | 8 | 7,0 | 6,0 | 6,0 |
| MF-Kapsel-Slurry mit Geruchsneutralisierer 1 (enthält 34% Geruchsneutralisierer 1) | 0,3 % | 7,5 | 5,0 | 4,0 | 2,0 |
| MF-Kapsel-Slurry mit Geruchsneutralisierer 2 (enthält 34% Geruchsneutralisierer 2) | 0,3 % | 8 | 6,0 | 4,0 | 2,5 |
| MF-Kapsel-Slurry mit Geruchsneutralisierer 3 (enthält 34% Geruchsneutralisierer 3) | 0,3 % | 8 | 6,5 | 5,0 | 4,0 |

| | | | | | |
|---|---|---|---|---|---|
| Geruchsneutralisierer 1: 3,5-Bis(2,4-dimethylcyclohex-3-en-1-yl)dihydro-1H-[1,3]oxazolo[3,4-c][1,3]oxazol Geruchsneutralisierer 2: 1H,3H,5H-Oxazolo[3,4-c]oxazol, 3,5-diheptyldihydro-7a-methyl-Geruchsneutralisierer 3: Zinkricinoleat | | | | | |

## Patentansprüche

1. Zusammensetzung umfassend Mikrokapseln umfassend mindestens einen Kern und eine Schale, wobei der Kern mindestens ein Geruchsneutralisierungsmittel enthält, ausgewählt aus
einer 1-Aza-3,7-dioxabicyclo[3.3.0]octan-Verbindung der allgemeinen Formel (II) 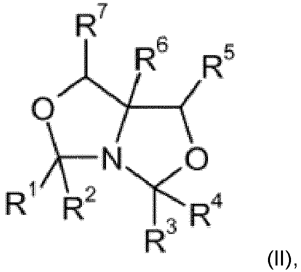
wobei, in den Verbindungen gemäß Formel (II), die Reste R¹, R², R³ und R⁴, jeweils unabhängig voneinander, für Reste stehen, die in einer Verbindung der allgemeinen Formel R¹-C(=O)-R² oder R³-C(=O)-R⁴ einen Duftstoff-Aldehyd mit mindestens 6 Kohlenstoffatomen oder ein Duftstoff-Keton mit mindestens 6 Kohlenstoffatomen ergeben, und die Reste R⁵, R⁶ und R⁷, jeweils unabhängig voneinander, für H oder einen Kohlenwasserstoffrest stehen, der acyclisch oder cyclisch, substituiert oder unsubstituiert, verzweigt oder unverzweigt, gesättigt oder ungesättift sein kann;
**dadurch gekennzeichnet, dass**, in den Verbindungen der Formel (II),
- die Reste R², R⁴, R⁵, und R⁷ für H stehen und die Reste R¹ und R³ jeweils unabhängig für einen C₆₋₂₄-Kohlenwasserstoffrest stehen und R⁶ für H oder einen C₁₋₂₄-Kohlenwasserstoffrest steht; oder
- die Reste R⁵ und R⁷, jeweils unabhängig voneinander, für Wasserstoff oder einen C₁₋₆-Kohlenwasserstoffrest, der ggf. substituiert sein kann, vorzugsweise C₁₋₃- Kohlenwasserstoffrest stehen, insbesondere sind R⁵ und R⁷ jeweils Wasserstoff oder jeweils ein Methyl- oder Ethylrest; und/oder
- der Rest R⁶ für H, einen Methyl-, Ethyl- oder Hydroxymethylrest steht; und/oder
- die Reste R² und R⁴ jeweils für H stehen; und/oder
- die Reste R², R⁴, R⁵ und R⁷ jeweils für H stehen, der Rest R⁶ für H, einen Methyl-, Ethyl- oder Hydroxymethylrest steht, die Reste R¹ und R³, jeweils unabhängig voneinander, jeweils für einen C₆₋₂₄-Kohlenwasserstoffrest, vorzugsweise C₇₋₂₄-Kohlenwasserstoffrest stehen, wobei der Kohlenwasserstoffrest acyclisch oder cyclisch, substituiert oder unsubstituiert, verzweigt oder unverzweigt, gesättigt oder ungesättigt sein kann; oder
die Reste R², R⁴, R⁵, R⁶ und R⁷ für H stehen und die Reste R¹ und R³ jeweils unabhängig für einen C₅₋₂₄-Kohlenwasserstoffrest stehen.

2. Die Zusammensetzung gemäß Anspruch 1, wobei die Mikrokapseln frei von zusätzlichem freien Duftstoff sind.

3. Die Zusammensetzung gemäß einem der Ansprüche 1 bis 2, **dadurch gekennzeichnet, dass**
- in den Verbindungen der Formel (II), die Reste R¹, R², R³ und R⁴ in einer Verbindung der allgemeinen Formel R¹-C(=O)-R² oder R³-C(=O)-R⁴ einen Duftstoffaldehyd oder ein Duftstoffketon ergeben, wobei der Duftstoffaldehyd ausgewählt ist aus der Gruppe bestehend aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-Isopropylphenyl)butanal), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), 4-Methoxybenzaldehyd, Benzaldehyd, 3-(4-tert-Butylphenyl)-propanal, 2-Methyl-3-(para-methoxyphenyl)propanal, 2-Methyl-4-(2,6,6-trimethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methyl-phenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethyl-cyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pen-tenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexen-carboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-unde-cadien-1-al), Hexahydro- 4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal;
und/oder das Duftstoffketon ausgewählt ist aus der Gruppe bestehend aus Methyl-beta-naphthylketon, Moschusindanon (1,2,3,5,6,7-Hexahydro-1,1,2,3,3-pentamethyl-4H-inden-4-on), Tonalid (6-Acetyl-1,1,2,4,4,7-hexamethyltetralin ), alpha-Damascone, beta-Damascone, delta-Damascone, iso-Damascone, Damascenone, Methyldihydrojasmonat, Menthon, Carvon, Kampfer, Koavon (3,4,5,6,6-Pentamethylhept-3-en-2-on), Fenchon, alpha-lonon, beta-lonon, gamma-Methyl-lonon, Fleuramon (2-Heptylcyclopentanon), Dihydrojasmon, cis-Jasmon, iso-E-Super (1-(1,2,3,4,5,6,7,8-Octahydro-2,3,8,8-tetramethyl-2-naphthalenyl)-ethan-1-on (und Isomere)), Methylcedrenylketon, Acetophenon, Methylacetophenon, para-Methoxyacetophenon, Methyl-beta-naphtylketon, Benzylaceton, Benzophenon, para-Hydroxyphenylbutanon, Sellerie-Keton(3- methyl-5-propyl-2-cyclohexenon), 6-Isopropyldecahydro-2-naphton, Dimethyloctenon, Frescomenthe (2-Butan-2-yl-cyclohexan-1-on), 4-(1-Ethoxyvinyl)-3,3,5,5-tetramethylcyclohexanon, Methylheptenon, 2-(2-(4-Methyl-3-cyclohexen-1-yl)propyl)-cyclopentanon, 1-(p-Menthen-6(2)yl)-1-propanon, 4-(4-Hydroxy-3-meth-oxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl (4-(1,3-Benzodioxol-5-yl) butan-2-on), Hexalon (1-(2,6,6-Trimethyl-2-cyclohexene-1-yl)-1,6-heptadien-3-on), Isocyclemon E (2-Acetonaphthon-1,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541-91-3), Neobutenon (1-(5,5-Dimethyl-1-cyclohexenyl)pent-4-en-1-on), Plicaton (CAS 41724-19-0), Velouton (2,2,5-Trimethyl-5-pentyl-cyclopentan-1-on), 2,4,4,7-Tetramethyl-oct-6-en-3-on sowie Tetrameran (6,10-Dimethylundecen-2-on); und/oder
der Kern der Mikrokapseln des Weiteren mindestens ein Duftstoffaldehyd und/oder Duftstoffketon umfasst, jeweils vorzugsweise ausgewählt aus der Gruppe der vorstehend genannten Duftstoffaldehyde und Duftstoffketone.

4. Die Zusammensetzung gemäß einem der Ansprüche 1 bis 3, **dadurch gekennzeichnet, dass** der Kern der Mikrokapseln des Weiteren mindestens einen Kieselsäureester der allgemeinen Formel (III) umfasst: 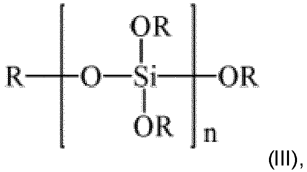 wobei, in den Verbindungen gemäß Formel (III), alle R jeweils unabhängig voneinander ausgewählt sind aus der Gruppe bestehend aus H, geradkettigen oder verzweigten, gesättigten oder ungesättigten, substituierten oder unsubstituierten C₁-C₆-Kohlenwasserstoffresten und Duftstoffalkoholresten; und n Werte aus dem Bereich 2 bis 100 annimmt.

5. Die Zusammensetzung gemäß Anspruch 4, **dadurch gekennzeichnet, dass**, in den Verbindungen der Formel (III),
- ein Teil der Reste R, vorzugsweise mindestens 5 mol-% der Reste R, ausgewählt ist aus der Gruppe bestehend aus Methyl, Ethyl, n-Propyl, iso-Propyl, n-Butyl, iso-Butyl und tert-Butyl; und/oder
- mindestens 10 mol-%, vorzugsweise mindestens 20 mol-% und insbesondere bevorzugt sogar mehr als 40 mol-% der Reste R ausgewählt sind aus der Gruppe bestehend aus den Resten der Duftstoffalkohole 10-Undecen-1-ol, 2,6- Dimethylheptan-2-ol, 2-Methyl-butanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl-pentanol, 3-Octanol, 3-Phenylpropanol, 4-Heptenol, 4-Isopropylcyclohexanol, 4-tert.-Butycyclohexanol, 6,8-Dimethyl-2-nonanol, 6-Nonen-1-ol, 9-Decen-1-ol, α-Methylbenzylalkohol, α-Terpineol, Amylsalicylat, Benzylalkohol, Benzylsalicylat, β-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanillin, Eugenol, Farnesol, Geraniol, Heptanol, Hexylsalicylat, Isoborneol, Isoeugenol, Isopulegol, Linalool, Menthol, Myrtenol, n-Hexanol, Nerol, Nonanol, Octanol, p-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Champiniol, Hexenol, Zimtalkohol und Mischungen daraus; und/oder
- n Werte aus dem Bereich von 2 bis 50, vorzugsweise aus dem Bereich 2 bis 20 und insbesondere aus dem Bereich von 3 bis 10 unter besonderer Bevorzugung der Werte 4, 5, 6, 7 und 8 annimmt.

6. Die Zusammensetzung gemäß einem der Ansprüche 1 bis 5, **dadurch gekennzeichnet, dass**
- es sich bei der Zusammensetzung um ein Wasch-, Reinigungs- oder Behandlungsmitteladditiv handelt; und/oder
- die Zusammensetzung mindestens ein weiteres Vorteilsmittel enthält; und/oder
- das mindestens eine Geruchsneutralisierungsmittel in einer Menge von ungefähr 0,1 bis ungefähr 40 Gew.-%, vorzugsweise ungefähr 0,1 bis ungefähr 20 Gew.-%, jeweils bezogen auf das Gesamtgewicht der Zusammensetzung, in dieser enthalten ist; und/oder
- der mindestens eine Kieselsäureester in einer Menge von ungefähr 0,1 bis ungefähr 40 Gew.-%, vorzugsweise ungefähr 0,1 bis ungefähr 20 Gew.-%, jeweils bezogen auf das Gesamtgewicht der Zusammensetzung, in dieser enthalten ist; und/oder.

7. Die Zusammensetzung gemäß einem der Ansprüche 1 bis 6, **dadurch gekennzeichnet, dass**
- die Mikrokapseln in einer Menge von ungefähr 0,01 bis 50 Gew.-%, vorzugsweise ungefähr 0,1 bis 25 Gew.-%, insbesondere 0,1 bis 10 Gew.-% enthalten sind, jeweils bezogen auf das Gesamtgewicht der Zusammensetzung; und/oder
- die Mikrokapseln in Form eines Kapsel-Slurries enthalten sind.

8. Mittel umfassend mindestens eine Zusammensetzung gemäß einem der Ansprüche 1 bis 7, wobei es sich bei dem Mittel um ein Wasch-, Reinigungs- oder Behandlungsmittel handelt.

9. Das Mittel gemäß Anspruch 8, **dadurch gekennzeichnet, dass** die mindestens eine Zusammensetzung gemäß einem der Ansprüche 1 bis 8 in einer Menge von bis zu ungefähr 30 Gew.-%, vorzugsweise bis zu ungefähr 20 Gew.-%, noch bevorzugter bis zu ungefähr 15 Gew.-% enthalten ist.

10. Das Mittel gemäß Anspruch 8 oder Anspruch 9, **dadurch gekennzeichnet, dass** es des Weiteren mindestens eine Komponente ausgewählt aus Enzymen, Tensiden, Bleichmitteln, Komplexbildnern, Gerüststoffen, Elektrolyten, nichtwässrigen Lösungsmitteln, pH-Stellmitteln, weiteren Duftstoffen, weiteren Duftstoffträgern, Fluoreszenzmitteln, Farbstoffen, Hydrotropen, Schauminhibitoren, Silikonölen, Antiredepositionsmitteln, Vergrauungsinhibitoren, Einlaufverhinderern, Knitterschutzmitteln, Farbübertragungsinhibitoren, antimikrobiellen Wirkstoffen, Germiziden, Fungiziden, Antioxidantien, Korrosionsinhibitoren, Antistatika, Bittermitteln, Bügelhilfsmitteln, Phobier- und Imprägniermitteln, Quell- und Schiebefestmitteln, weichmachenden Komponenten sowie UV-Absorbern umfasst.

11. Verwendung einer Zusammensetzung gemäß einem der Ansprüche 1 bis 7 oder eines Mittels gemäß einem der Ansprüche 8 bis 10 zur Verminderung von Fehlgerüchen.

12. Verfahren zur Behandlung einer Oberfläche, zur Behandlung der Raumluft, oder zum Waschen und/oder Pflegen von Textilien, **dadurch gekennzeichnet, dass** in mindestens einem Verfahrensschritt eine Zusammensetzung gemäß einem der Ansprüche 1 bis 7 und/oder ein Mittel gemäß einem der Ansprüche 8 bis 10 angewendet wird.

## Claims

1. A composition comprising microcapsules comprising at least one core and one shell, wherein the core contains at least one odor-neutralizing agent selected from a 1-aza-3,7-dioxabicyclo[3.3.0]octane compound of general formula (II) 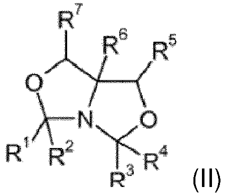
where, in the compounds according to formula (II), the functional groups R¹, R², R³ and R⁴ each independently represent functional groups which, in a compound of the general formula R¹-C(=O)-R² or R³-C(=O)-R⁴, produce a fragrance aldehyde having at least 6 carbon atoms or a fragrance ketone having at least 6 carbon atoms, and the functional groups R⁵, R⁶ and R⁷ each independently represent H or a hydrocarbon functional group which can be acyclic or cyclic, substituted or unsubstituted, branched or unbranched, saturated or unsaturated;
**characterized in that,** in the compounds of formula (II),
- the functional groups R², R⁴, R⁵, and R⁷represent H and the functional groups R¹and R³ each independently represent a C₆₋₂₄ hydrocarbon functional group and R⁶ represents H or a C₁₋₂₄ hydrocarbon functional group; or
- the functional groups R⁵and R⁷ each independently represent hydrogen or a C₁₋₆ hydrocarbon functional group which may optionally be substituted, preferably a C₁₋₃ hydrocarbon functional group, in particular R⁵ and R⁷ are in each case hydrogen or a methyl or ethyl functional group; and/or
- R⁶ represents H, a methyl, ethyl or hydroxymethyl functional group; and/or
- the functional groups R² and R⁴ each represent H; and/or
- the functional groups R², R⁴, R⁵ and R⁷ each represent H, R⁶ represents H, a methyl, ethyl or hydroxymethyl functional group, R¹ and R³ each independently represent a C₆₋₂₄ hydrocarbon functional group, preferably a C₇₋₂₄ hydrocarbon functional group, wherein the hydrocarbon functional group can be acyclic or cyclic, substituted or unsubstituted, branched or unbranched, saturated or unsaturated; or
the functional groups R², R⁴, R⁵, R⁶ and R⁷ represent H; and the functional groups R¹ and R³ each independently represent a C₅₋₂₄ hydrocarbon functional group.

2. The composition according to claim 1, wherein the microcapsules are free of additional free fragrance.

3. The composition according to one of claims 1 to 2, **characterized in that,** in the compounds of formula (II), the functional groups R¹, R², R³ and R⁴ in a compound of the general formula R¹-C(=O)-R² or R³-C(=O)-R⁴ produce a fragrance aldehyde or a fragrance ketone, wherein the fragrance aldehyde is selected from the group consisting of Adoxal (2,6,10-trimethyl-9-undecenal), anisaldehyde (4-methoxybenzaldehyde), cymene (3-(4-isopropylphenyl)-2-methylpropanal), ethylvanillin, Florhydral (3-(3-isopropylphenyl)butanal), helional (3-(3,4-methylenedioxyphenyl)-2-methylpropanal), heliotropin, hydroxycitronellal, lauraldehyde, Lyral (3- and 4-(4-hydroxy-4-methylpentyl)-3-cyclohexene-1-carboxaldehyde), methylnonylacetaldehyde, lilial (3-(4-tert-butylphenyl)-2-methylpropanal), phenylacetaldehyde, undecylenealdehyde, vanillin, 2,6,10-trimethyl-9-undecenal, 3-dodecen-1-al, alpha-n-amylcinnamaldehyde, melonal (2,6-dimethyl-5-heptenal), 2,4-dimethyl-3-cyclohexene-1-carboxaldehyde (Triplal), 4-methoxybenzaldehyde, benzaldehyde, 3-(4-tert-butylphenyl)-propanal, 2-methyl-3-(para-methoxyphenyl)propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decylidene-8)-butanal, octahydro-4,7-methane-1H-indenecarboxaldehyde, 3-ethoxy-4-hydroxybenzaldehyde, para-ethyl-alpha,alpha-dimethylhydrocinnamaldehyde, alpha-methyl-3,4-(methylenedioxy)-hydrocinnamaldehyde, 3,4-methylenedioxybenzaldehyde, alpha-n-hexylcinnamaldehyde, m-cymene-7-carboxaldehyde, alpha-methylphenylacetaldehyde, 7-hydroxy-3,7-dimethyloctanal, undecenal, 2,4,6-trimethyl -3-cyclohexene-1-carboxaldehyde, 4-(3)(4-methyl-3-pentenyl)-3-cyclohexenecarboxaldehyde, 1-dodecanal, 2,4-dimethyl-cyclohexene-3-carboxaldehyde, 4-(4-hydroxy-4-methylpentyl)-3-cyclohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanindan-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyl-octanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanindan-1-carboxaldehyde, 2-methyloctanal, alpha-methyl-4-(1-methylethyl)benzeneacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propyl-bicyclo[2.2.1]-hept-5-ene-2-carbaldehyde, 9-decenal, 3-methyl-5-phenyl-1-pentanal, methylnonylacetaldehyde, hexanal and trans-2-hexenal;
and/or the fragrance ketone is selected from the group consisting of methyl-beta-naphthyl ketone, musk indanone (1,2,3,5,6,7-hexahydro-1,1,2,3,3-pentamethyl-4H-inden-4-one), tonalide (6-acetyl-1,1,2,4,4,7-hexamethyltetralin), alpha-damascone, beta-damascone, delta-damascone, iso-damascone, damascenone, methyldihydrojasmonate, menthone, carvone, camphor, Koavone (3,4,5,6,6-pentamethylhept-3-en-2-one), fenchone, alpha-ionone, beta-ionone, gamma-methyl-ionone, fleuramone (2-heptylcyclopentanone), dihydrojasmone, cis-jasmone, Iso-E-Super (1-(1,2,3,4,5,6,7,8-octahydro-2,3,8,8-tetramethyl-2-naphthalenyl)-ethan-1-one (and isomers)), methyl cedrenyl ketone, acetophenone, methylacetophenone, para-methoxy acetophenone, methyl beta-naphthyl ketone, benzyl acetone, benzophenone, para-hydroxyphenyl butanone, celery ketone (3-methyl-5-propyl-2-cyclohexenone), 6-isopropyldecahydro-2-naphthone, dimethyloctenone, Frescomenthe (2-butan-2-yl-cyclohexan-1-one), 4-(1-ethoxyvinyl)-3,3,5,5-tetramethylcyclohexanone, methylheptenone, 2-(2-(4-methyl-3-cyclohexen-1-yl)propyl)-cyclopentanone,1-(p-menthen-6(2)-yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)-indanone, 4-damascol, Dulcinyl (4-(1,3-benzodioxol-5-yl)butan-2-one), Hexalone (1-(2,6,6-trimethyl-2-cyclohexen-1-yl)-1,6-heptadien-3-one), Isocyclemone E (2-acetonaphthone-1,2,3,4,5,6,7,8-octahydro-2,3,8,8-tetramethyl), methyl nonyl ketone, methylcyclocitrone, methyl lavender ketone, Orivone (4-tert-amyl-cyclohexanone), 4-tert-butylcyclohexanone, Delphone (2-pentyl-cyclopentanone), muscone (CAS 541-91-3), Neobutenone (1-(5,5-dimethyl-1-cyclohexenyl)pent-4-en-1-one), plicatone (CAS 41724-19-0), Veloutone (2,2,5-trimethyl-5-pentylcyclopentan-1-one), 2,4,4,7-tetramethyl-oct-6-en-3-one and tetramerane (6,10-dimethylundecen-2-one); and/or the core of the microcapsules further comprises at least one fragrance aldehyde and/or fragrance ketone, each preferably selected from the group of the above-mentioned fragrance aldehydes and fragrance ketones.

4. The composition according to one of claims 1 to 3, **characterized in that** the core of the microcapsules further comprises at least one silicic acid ester of general formula (III): 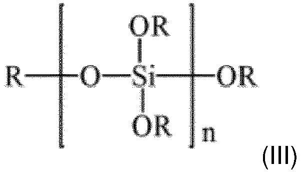 where, in the compounds according to formula (III), all R are each independently selected from the group consisting of H, straight-chain or branched, saturated or unsaturated, substituted or unsubstituted C₁-C₆ hydrocarbon functional groups and fragrance alcohol functional groups; and n assumes values in the range 2 to 100.

5. The composition according to claim 4, **characterized in that,** in the compounds of formula (III),
- some of the R functional groups, preferably at least 5 mol.% of the functional groups R, are selected from the group consisting of methyl, ethyl, n-propyl, iso-propyl, n-butyl, iso-butyl and tert-butyl; and/or
- at least 10 mol.%, preferably at least 20 mol.% and in particular preferably even more than 40 mol.% of the functional groups R are selected from the group consisting of the functional groups of the fragrance alcohols 10-undecen-1-ol,2,6-dimethylheptan-2-ol, 2-methylbutanol, 2-methylpentanol, 2-phenoxyethanol, 2-phenylpropanol, 2-tert-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-tert-butylcyclohexanol, 6,8-dimethyl-2-nonanol, 6-nonen-1-ol,9-decen-1-ol, α-methyl benzyl alcohol, α-terpineol, amyl salicylate, benzyl alcohol, benzyl salicylate, β-terpineol, butyl salicylate, citronellol, cyclohexyl salicylate, decanol, dihydromyrcenol, dimethyl benzyl carbinol, dimethyl heptanol, dimethyl octanol, ethyl salicylate, ethylvanillin, eugenol, farnesol, geraniol, heptanol, hexyl salicylate, isoborneol, isoeugenol, isopulegol, linalool, menthol, myrtenol, n-hexanol, nerol, nonanol, octanol, p-menthan-7-ol, phenylethyl alcohol, phenol, phenyl salicylate, tetrahydrogeraniol, tetrahydrolinalool, thymol, trans-2-cis-6-nonadicnol, trans-2-nonen-1-ol, trans-2-octenol, undecanol, vanillin, champiniol, hexenol, cinnamic alcohol, and mixtures thereof; and/or
- n assumes values in the range of from 2 to 50, preferably in the range 2 to 20 and in particular in the range of from 3 to 10, with particular preference for the values 4, 5, 6, 7 and 8.

6. The composition according to one of claims 1 to 5, **characterized in that**
- the composition is a washing, cleaning, or treatment agent additive; and/or
- the composition contains at least one further benefit agent; and/or
- the at least one odor-neutralizing agent is contained in the composition in an amount of from approximately 0.1 to approximately 40 wt.%, preferably from approximately 0.1 to approximately 20 wt.%, each based on the total weight of the composition; and/or
- the at least one silicic acid ester is contained in the composition in an amount of from approximately 0.1 to approximately 40 wt.%, preferably approximately 0.1 to approximately 20 wt.%, in each case based on the total weight of the composition; and/or.

7. The composition according to one of claims 1 to 6, **characterized in that**
- the microcapsules are present in an amount of from approximately 0.01 to 50 wt.%, preferably approximately 0.1 to 25 wt.%, in particular 0.1 to 10 wt.%, in each case based on the total weight of the composition; and/or
- the microcapsules are contained in the form of a capsule slurry.

8. An agent comprising at least one composition according to one of claims 1 to 7, wherein the agent is a washing agent, cleaning agent or treatment agent.

9. The agent according to claim 8, **characterized in that** the at least one composition according to one of claims 1 to 8 is contained in an amount of up to approximately 30 wt.%, preferably up to approximately 20 wt.%, more preferably up to approximately 15 wt.%.

10. The agent according to claim 8 or claim 9, **characterized in that** it further comprises at least one component selected from enzymes, surfactants, bleaching agents, complexing agents, builders, electrolytes, non-aqueous solvents, pH adjusters, additional fragrances, additional fragrance carriers, fluorescing agents, dyes, hydrotropic substances, foam inhibitors, silicone oils, anti-redeposition agents, graying inhibitors, shrinkage inhibitors, anti-crease agents, dye transfer inhibitors, antimicrobial active ingredients, germicides, fungicides, antioxidants, corrosion inhibitors, antistatic agents, bittering agents, ironing aids, repellents and impregnating agents, swelling and non-slip agents, softening components and UV absorbers.

11. The use of a composition according to one of claims 1 to 7 or of an agent according to one of claims 8 to 10 for reducing malodors.

12. A method for treating a surface, for treating room air, or for washing and/or caring for textiles, **characterized in that** a composition according to one of claims 1 to 7 and/or an agent according to one of claims 8 to 10 is applied in at least one method step.

## Revendications

1. Composition comprenant des microcapsules comprenant au moins un noyau et une enveloppe, dans laquelle le noyau contient au moins un agent neutralisateur d'odeur, choisi parmi un composé 1-aza-3,7-dioxabicyclo[3.3.0]octane de formule générale (II) 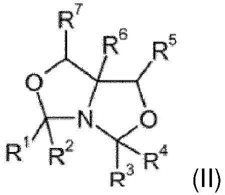
dans laquelle, dans les composés selon la formule (II), les radicaux R¹, R², R³ et R⁴ représentent, respectivement indépendamment les uns des autres, des radicaux qui, dans un composé de formule générale R¹-C(=O)-R² ou R³-C(=O)-R⁴, donnent un aldéhyde de parfum comportant au moins 6 atomes de carbone ou une cétone de parfum comportant au moins 6 atomes de carbone, et les radicaux R⁵, R⁶ et R⁷ représentent, respectivement indépendamment les uns des autres, H ou un radical hydrocarboné, qui peut être acyclique ou cyclique, substitué ou non substitué, ramifié ou non ramifié, saturé ou insaturé ;
**caractérisée en ce que,** dans les composés de formule (II),
- les radicaux R², R⁴, R⁵ et R⁷ représentent H et les radicaux R¹ et R³ représentent respectivement indépendamment un radical hydrocarboné en C₆₋₂₄ et R⁶ représente H ou un radical hydrocarboné en C₁₋₂₄ ; ou
- les radicaux R⁵ et R⁷ représentent, respectivement indépendamment l'un de l'autre, l'hydrogène ou un radical hydrocarboné en C₁₋₆, qui peut éventuellement être substitué, de préférence un radical hydrocarboné en C₁₋₃, en particulier R⁵ et R⁷ représentent respectivement l'hydrogène ou respectivement un radical méthyle ou éthyle ; et/ou
- le radical R⁶ représente H, un radical méthyle, éthyle ou hydroxyméthyle ; et/ou
- les radicaux R² et R⁴ représentent respectivement H ; et/ou
- les radicaux R², R⁴, R⁵ et R⁷ représentent respectivement H, le radical R⁶ représente H, un radical méthyle, éthyle ou hydroxyméthyle, les radicaux R¹ et R³ représentent, respectivement indépendamment l'un de l'autre, respectivement un radical hydrocarboné en C₆₋₂₄, de préférence un radical hydrocarboné en C₇₋₂₄, dans laquelle le radical hydrocarboné peut être acyclique ou cyclique, substitué ou non substitué, ramifié ou non ramifié, saturé ou insaturé ; ou
les radicaux R², R⁴, R⁵, R⁶ et R⁷ représentent H et les radicaux R¹ et R³ représentent respectivement indépendamment un radical hydrocarboné en C₅₋₂₄.

2. Composition selon la revendication 1, dans laquelle les microcapsules sont exemptes de parfum libre supplémentaire.

3. Composition selon l'une des revendications 1 à 2, **caractérisée en ce que**
- dans les composés de formule (II), les radicaux R¹, R², R³ et R⁴ donnent, dans un composé de formule générale R¹-C(=O)-R² ou R³-C(=O)-R⁴, un aldéhyde de parfum ou une cétone de parfum, dans laquelle l'aldéhyde de parfum est choisi dans le groupe constitué d'Adoxal (2,6,10-triméthyl-9-undécénal), anisaldéhyde (4-méthoxybenzaldéhyde), cymal (3-(4-isopropylphényl)-2-méthylpropanal), éthylvanilline, Florhydral (3-(3-isopropylphényl)butanal), Helional (3-(3,4-méthylènedioxyphényl)-2-méthylpropanal), héliotropine, hydroxycitronellal, lauraldéhyde, Lyral (3- et 4-(4-hydroxy-4-méthylpentyl)-3-cyclohexène-1-carboxaldéhyde), méthylnonylacétaldéhyde, Lilial (3-(4-tert-butylphényl)-2-méthylpropanal), phénylacétaldéhyde, undécylènealdéhyde, vanilline, 2,6,10-triméthyl-9-undécénal, 3-dodécén-1-al, alpha-n-amylcinnamaldéhyde, Melonal (2,6-diméthyl-5-hepténal), 2,4-diméthyl-3-cyclohexène-1-carboxaldéhyde (Triplal), 4-méthoxybenzaldéhyde, benzaldéhyde, 3-(4-tert-butylphényl)-propanal, 2-méthyl-3-(para-méthoxyphényl)propanal, 2-méthyl-4-(2,6,6-triméthyl-2(1)-cyclohexén-1-yl)butanal, 3-phényl-2-propénal, cis-/trans-3,7-diméthyl-2,6-octadién-1-al, 3,7-diméthyl-6-octén-1-al, [(3,7-diméthyl-6-octényl)oxy]acétaldéhyde, 4-isopropylbenzylaldéhyde, 1,2,3,4,5,6,7,8-octahydro-8,8-diméthyl-2-naphtaldéhyde, 2,4-diméthyl-3-cyclohexène-1-carboxaldéhyde,2-méthyl-3-(isopropylphényl)propanal, 1-décanal, 2,6-diméthyl-5-hepténal, 4-(tricyclo[5.2.1.0(2,6)]-décylidène-8)-butanal, octahydro-4,7-méthane-1H-indènecarboxaldéhyde, 3-éthoxy-4-hydroxybenzaldéhyde, para-éthyl-alpha,alpha-diméthylhydrocinnamaldéhyde, alpha-méthyl-3,4-(méthylènedioxy)-hydrocinnamaldéhyde, 3,4-méthylènedioxybenzaldéhyde, alpha-n-hexylcinnamaldéhyde, m-cymène-7-carboxaldéhyde, alpha-méthylphénylacétaldéhyde, 7-hydroxy-3,7-diméthyloctanal, undécénal, 2,4,6-triméthyl-3-cyclohexène-1-carboxaldéhyde, 4-(3)(4-méthyl-3-pentényle)-3-cyclohexènecarboxaldéhyde, 1-dodécanal, 2,4-diméthyl-cyclohexène-3-carboxaldéhyde, 4-(4-hydroxy-4-méthylpentyl)-3-cyclohexène-1-carboxaldéhyde, 7-méthoxy-3,7-diméthyloctan-1-al, 2-méthylundécanal, 2-méthyldécanal, 1-nonanal, 1-octanal, 2,6,10-triméthyl-5,9-undécadiénal, 2-méthyl-3-(4-tert-butyl)propanal, dihydrocinnamaldéhyde, 1-méthyl-4-(4-méthyl-3-pen-tényl)-3-cyclohexène-1-carboxaldéhyde, 5- ou 6-méthoxyhexahydro-4,7-méthanindane-1- ou -2-carboxaldéhyde, 3,7-diméthyloctan-1-al, 1-undécanal, 10-undécén-1-al, 4-hydroxy-3-méthoxybenzaldéhyde, 1-méthyl-3-(4-méthylpentyl)-3-cyclohexènecarboxaldéhyde, 7-hydroxy-3,7-diméthyl-octanal, trans-4-décénal, 2,6-nonadiénal, para-tolylacétaldéhyde, 4-méthylphénylacétaldéhyde, 2-méthyl-4-(2,6,6-triméthyl-1 -cyclohexén-1 -yl)-2-buténal, ortho-méthoxycinnamaldéhyde, 3,5,6-triméthyl-3-cyclohexènecarboxaldéhyde, 3,7-diméthyl-2-méthylén-6-octénal, phénoxyacétaldéhyde, 5,9-diméthyl-4,8-décadiénal, aldéhyde de pivoine (6,10-diméthyl-3-oxa-5,9-undé-cadién-1-al), hexahydro-4,7-méthanindane-1-carboxaldéhyde, 2-méthyloctanal, alpha-méthyl-4-(1-méthyléthyl)benzèneacétaldéhyde, 6,6-diméthyl-2-norpinène-2-propionaldéhyde, para-méthylphénoxyacétaldéhyde, 2-méthyl-3-phényl-2-propén-1-al, 3,5,5-triméthylhexanal, hexahydro-8,8-diméthyl-2-naphtaldéhyde, 3-propyl-bicyclo[2.2.1]-hept-5-ène-2-carbaldéhyde, 9-décénal, 3-méthyl-5-phényl-1-pentanal, méthylnonylacétaldéhyde, hexanal ainsi que trans-2-hexénal ;
et/ou la cétone de parfum est choisie dans le groupe constitué de méthyl-bêta-naphtylcétone, indanone de musc (1,2,3,5,6,7-hexahydro-1,1,2,3,3-pentaméthyl-4H-indén-4-one), Tonalide (6-acétyl-1,1,2,4,4,7-hexaméthyltétraline), alpha-damascone, bêta-damascone, delta-damascone, iso-damascone, damascénone, méthyldihydrojasmonate, menthone, carvone, camphre, Koavone (3,4,5,6,6-pentaméthylhept-3-én-2-one), fenchone, alpha-ionone, bêta-ionone, gamma-méthyl-ionone, fleuramone (2-heptylcyclopentanone), dihydrojasmone, cis-jasmone, iso-E-Super (1-(1,2,3,4,5,6,7,8-octahydro-2,3,8,8-tétraméthyl-2-naphtalényl)-éthan-1-one (et isomères), méthylcédrénylcétone, acétophénone, méthylacétophénone, para-méthoxyacétophénone, méthyl-bêta-naphtylcétone, benzylacétone, benzophénone, para-hydroxyphénylbutanone, céleri-cétone(3-méthyl-5-propyl-2-cyclohexénone), 6-isopropyldécahydro-2-naphtone, diméthylocténone, Frescomenthe (2-butan-2-yl-cyclohexan-1 -one), 4-(1-éthoxyvinyl)-3,3,5,5-tétraméthylcyclohexanone, méthylhepténone, 2-(2-(4-méthyl-3-cyclohexén-1 -yl)propyl)-cyclopentanone, 1-(p-menthén-6(2)yl)-1-propanone, 4-(4-hydroxy-3-méth-oxyphényl)-2-butanone, 2-acétyl-3,3-diméthylnorbornane, 6,7-dihydro-1,1,2,3,3-pentaméthyl-4(5H)-indanone, 4-damascol, Dulcinyl (4-(1,3-benzodioxol-5-yl)butan-2-one), Hexalone (1-(2,6,6-triméthyl-2-cyclohexén-1-yl)-1,6-heptadién-3-one), isocyclémone E (2-acétonaphton-1,2,3,4,5,6,7,8-octahydro-2,3,8,8-tétraméthyle), méthylnonylcétone, méthylcyclocitrone, méthyllavandecétone, Orivone (4-tert-amylcyclohexanone), 4-tert-butylcyclohexanone, delphone (2-pentylcyclopentanone), muscone (CAS 541-91-3), néobuténone (1-(5,5-diméthyl-1-cyclohexényl)pent-4-én-1-one), plicatone (CAS 41724-19-0), Veloutone (2,2,5-triméthyl-5-pentyl-cyclopentan-1-one), 2,4,4,7-tétraméthyl-oct-6-én-3-one ainsi que tétraméran (6,10-diméthylundécén-2-one) ; et/ou le noyau des microcapsules comprend en outre au moins un aldéhyde de parfum et/ou une cétone de parfum, respectivement de préférence choisis dans le groupe des aldéhydes de parfum et cétones de parfum susmentionnés.

4. Composition selon l'une des revendications 1 à 3,
**caractérisée en ce que** le noyau des microcapsules comprend en outre au moins un ester d'acide silicique de formule générale (III) : 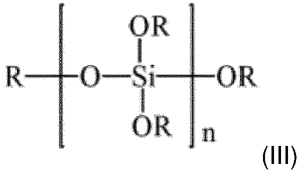 dans laquelle, dans les composés selon la formule (III), tous les R sont choisis respectivement indépendamment les uns des autres dans le groupe constitué de H, de radicaux hydrocarbonés en C₁-C₆ à chaîne droite ou ramifiés, saturés ou insaturés, substitués ou non substitués, et de radicaux d'alcool de parfum ; et n adopte des valeurs dans la plage allant de 2 à 100.

5. Composition selon la revendication 4, **caractérisée en ce que,** dans les composés de formule (III),
- une partie des radicaux R, de préférence au moins 5 % en moles des radicaux R, est choisie dans le groupe constitué de méthyle, éthyle, n-propyle, iso-propyle, n-butyle, iso-butyle et tert-butyle ; et/ou
- au moins 10 % en moles, de préférence au moins 20 % en moles et de manière particulièrement préférée plus de 40 % en moles des radicaux R sont choisis dans le groupe constitué des radicaux des alcools de parfum 10-undécén-1-ol, 2,6-diméthylheptan-2-ol, 2-méthyl-butanol, 2-méthylpentanol, 2-phénoxyéthanol, 2-phénylpropanol, 2-tert-butycyclohexanol, 3,5,5-triméthylcyclohexanol, 3-hexanol, 3-méthyl-5-phényl-pentanol, 3-octanol, 3-phénylpropanol, 4-hepténol, 4-isopropylcyclohexanol, 4-tert.-butycyclohexanol, 6,8-diméthyl-2-nonanol, 6-nonén-1-ol, 9-décén-1-ol, alcool a-méthylbenzylique, a-terpinéol, salicylate d'amyle, alcool benzylique, salicylate de benzyle, β-terpinéol, salicylate de butyle, citronellol, salicylate de cyclohexyle, décanol, dihydromyrcénol, diméthylbenzylcarbinol, diméthylheptanol, diméthyloctanol, salicylate d'éthyle, éthylvanilline, eugénol, farnésol, géraniol, heptanol, salicylate d'hexyle, isobornéol, isoeugénol, isopulégol, linalol, menthol, myrténol, n-hexanol, nérol, nonanol, octanol, p-menthan-7-ol, alcool phényléthylique, phénol, salicylate de phényle, tétrahydrogéraniol, tétrahydrolinalol, thymol, trans-2-cis-6-nonadicnol, trans-2-nonén-1-ol, trans-2-octénol, undécanol, vanilline, champiniol, hexénol, alcool cinnamylique et leurs mélanges ; et/ou
- n adopte des valeurs dans la plage allant de 2 à 50, de préférence dans la plage allant de 2 à 20 et en particulier dans la plage allant de 3 à 10 en donnant une préférence particulière pour les valeurs 4, 5, 6, 7 et 8.

6. Composition selon l'une des revendications 1 à 5, **caractérisée en ce que**
- la composition est un additif d'agent de lavage, de nettoyage ou de traitement ; et/ou
- la composition contient au moins un autre agent bénéfique ; et/ou
- l'au moins un agent neutralisateur d'odeur est contenu dans la composition en une quantité allant d'environ 0,1 à environ 40 % en poids, de préférence d'environ 0,1 à environ 20 % en poids, respectivement par rapport au poids total de ladite composition ; et/ou
- l'au moins un ester d'acide silicique est contenu dans la composition en une quantité allant d'environ 0,1 à environ 40 % en poids, de préférence d'environ 0,1 à environ 20 % en poids, respectivement par rapport au poids total de ladite composition ; et/ou.

7. Composition selon l'une des revendications 1 à 6, **caractérisée en ce que**
- les microcapsules sont contenues en une quantité allant d'environ 0,01 à 50 % en poids, de préférence d'environ 0,1 à 25 % en poids, en particulier de 0,1 à 10 % en poids, respectivement par rapport au poids total de la composition ; et/ou
- les microcapsules sont contenues sous la forme d'une suspension de capsules.

8. Agent comprenant au moins une composition selon l'une des revendications 1 à 7, dans lequel l'agent est un agent de lavage, de nettoyage ou de traitement.

9. Agent selon la revendication 8, **caractérisé en ce que** l'au moins une composition selon l'une des revendications 1 à 8 est contenue en une quantité allant jusqu'à environ 30 % en poids, de préférence jusqu'à environ 20 % en poids, plus préférablement jusqu'à environ 15 % en poids.

10. Agent selon la revendication 8 ou la revendication 9,
**caractérisé en ce qu'il** comprend en outre au moins un composant choisi parmi les enzymes, tensioactifs, agents de blanchiment, agents complexants, adjuvants, électrolytes, solvants non aqueux, agents régulateurs de pH, autres parfums, autres supports de parfum, agents fluorescents, colorants, hydrotropes, inhibiteurs de mousse, huiles de silicone, agents antiredéposition, inhibiteurs de grisaillement, agents antirétrécissement, agents antifroissage, inhibiteurs de transfert de couleur, principes actifs antimicrobiens, germicides, fongicides, antioxydants, inhibiteurs de corrosion, antistatiques, amérisants, auxiliaires de repassage, agents d'hydrofugation et d'imprégnation, agents empêchant le gonflement et l'éraillage, composants assouplissants ainsi qu'absorbeurs d'UV.

11. Utilisation d'une composition selon l'une des revendications 1 à 7 ou d'un agent selon l'une des revendications 8 à 10 pour réduire les mauvaises odeurs.

12. Procédé destiné au traitement d'une surface, au traitement de l'air ambiant ou au lavage et/ou à l'entretien de textiles, **caractérisé en ce que,** dans au moins une étape de procédé, une composition selon l'une des revendications 1 à 7 et/ou un agent selon l'une des revendications 8 à 10 sont utilisés.
